(19) Europäisches Patentamt
European Patent Office
Office européen des brevets

(11) Veröffentlichungsnummer : 0 299 313 B1

# (12) EUROPÄISCHE PATENTSCHRIFT

(45) Veröffentlichungstag der Patentschrift : 03.07.91 Patentblatt 91/27

(51) Int. Cl.[5] : A01N 37/34, C07C 255/30

(21) Anmeldenummer : 88110622.3

(22) Anmeldetag : 04.07.88

## (54) Schädlingsbekämpfungsmittel auf Basis von Derivaten des 2,3-Diaminomaleinsäurenitrils.

(30) Priorität : 17.07.87 DE 3723621

(43) Veröffentlichungstag der Anmeldung : 18.01.89 Patentblatt 89/03

(45) Bekanntmachung des Hinweises auf die Patenterteilung : 03.07.91 Patentblatt 91/27

(84) Benannte Vertragsstaaten : BE CH DE ES FR GB GR IT LI NL

(56) Entgegenhaltungen :
DE-A- 2 500 168
DE-A- 2 809 022
US-A- 4 002 616
THE JOURNAL OF ORGANIC CHEMISTRY, Band 39, Nr. 16, Mai-August 1974 R.W. BEGLAND et al. "Hydrogen Cyanide Chemistry. VIII. New Chemistry of Diaminomeleonitrile. Heterocyclic Synthesis" Seiten 2341-50

(56) Entgegenhaltungen :
CHEMICAL ABSTRACTS, Band 103, Nr. 1, 8 Juli 1985, Columbus, Ohio, USA O.MORIYA et al. "A convenient synthesis of 2-aryl-4, 5-dicyanoimidazoles and -imidazolines from diaminoameleonitrile." Seite 566, Spalte 2, Zusammenfassung-Nr. 6 273h
CHEMICAL ABSTRACTS, Band 88, Nr. 3, 16 .Jänner 1978, Columbus, Ohio, USA T. KOJIMA et al. "N-(Alkoxycarbonyl)diaminomaleonitriles." Seite 570, Spalte 2, Zusammenfassung-Nr.22 162y & Japan. Kokai 77,100,424
PATENT ABSTRACTS OF JAPAN, unexamined applications, Sektion C, Vol. 1, Nr. 29, 28 März 1977 THE PATENT OFFICE JAPANESE GOVERNMENT Seite 1678 C 76
THE JOURNAL OF THE AMERICAN CHEMICAL SOCIETY; Band LXXX, April-Juni 1958 PS ROBERTSON und J. VAUGHAN "Derivatives of the Hydrogen Cyanide Tetramer: Structure and Chemistry" Seiten 2691-2693

(73) Patentinhaber : BAYER AG
W-5090 Leverkusen 1 Bayerwerk (DE)

(72) Erfinder : Heidenreich, Holger, Dr.
Blankenese 18
W-2224-Kuden (DE)
Erfinder : Becker, Benedikt, Dr.
Metzkausener Strasse 14
W-4020 Mettmann (DE)

EP 0 299 313 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft die Verwendung von teilweise bekannten Derivaten von 2,3-Diaminomaleinsäurenitril zur Bekämpfung von Spinnentieren (Pflanzen-, Hygiene- und Vorratsschädlinge).

Derivate des 2,3-Diaminomaleinsäurenitrils sind aus verschiedenen Literaturstellen bekannt. Siehe z.B. US-Patentschrift Nr. 4 002 616, wo Bisanil-Derivate beschrieben werden oder siehe z.B. Onoda in Nippon Nogeikagaku Kaishi 36(2), 167-72 (1962), wo ebenso wie in den Publikationen Robertson, Vaughan in Journal of the American Chemical Society 80, 2691 (1958) und Hinkel et al., Journal of the Chemical Society 1937, 1432, Monoanilderivate von 2,3-Diaminomaleinsäurenitril beschrieben werden.

Aus folgenden Publikationen sind weiterhin ähnlich strukturierte Derivate des 2,3-Diaminomaleinsäurenitrils bekannt : DE-Al 2 809 022 ; DE-Al 2 500 168 ; Journal of Org.Chem. 39, Nr.16, S.2341,2342 (1974) ; Chem.Abstracts 103, 6 273 h (1985) ; Chem.Abstracts 88, 22 162 y (1978).

Über eine Wirksamkeit der vorgenannten Verbindungsklassen oder anderer Derivate des 2,3-Diaminomaleinsäurenitrils gegen Spinnentiere ist jedoch bisher noch nichts bekannt geworden.

Es wurde gefunden, daß die teilweise bekannten Derivate von 2,3-Diaminomaleinsäurenitril der allgemeinen Formel

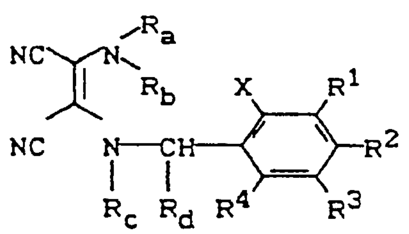

(I)

in welcher

X für Wasserstoff, Halogen, Halogenalkyl oder CN steht,

$R^1$, $R^2$, $R^3$ und $R^4$ gleich oder verschieden sein können und für Wasserstoff, gegebenenfalls durch Halogen, OH, $NH_2$ substituiertes ($C_1$-$C_4$)Alkyl, gegebenenfalls durch Halogen, OH, $NH_2$ substituiertes ($C_1$-$C_4$)Alkoxy, Halogen, CN, $NO_2$, gegebenenfalls durch Halogen, OH, $NH_2$ substituiertes Dialkyl ($C_1$-$C_4$)amino, Alkoxy($C_1$-$C_4$)carbonyl, gegebenenfalls durch Halogen, OH, $NH_2$ substituiertes Alkyl($C_1$ -$C_4$)thio, gegebenenfalls durch Halogen, OH, $NH_2$ substituiertes Alkyl($C_1$-$C_4$)sulfonyl, OH, SH, $NH_2$ stehen,

und die Substituenten $R_a$, $R_b$, $R_c$ und $R_d$ jeweils eine der folgenden Bedeutungskomninationen A, B, C oder D annehmen können :

A) $R_a$ und $R_b$ stehen zusammen für den Rest

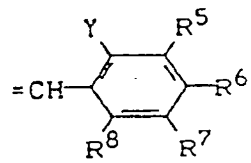

wobei

Y und $R^8$ gleich oder verschieden sein können und für Wasserstoff, Fluor, Chlor, Brom, Jod, $CF_3$ oder CN steht,

$R^5$, $R^6$ und $R^7$ gleich oder verschieden sein können und für Wasserstoff, gegebenenfalls durch Halogen, OH, $NH_2$ substituiertes ($C_1$-$C_4$)Alkyl, gegebenenfalls durch Halogen, OH, $NH_2$ substituiertes ($C_1$-$C_4$)Alkoxy, Halogen, CN, $NO_2$, gegebenenfalls durch Halogen, OH, $NH_2$ substituiertes Dialkyl($C_1$-$C_4$)amino, Alkoxy($C_1$-$C_4$)carbonyl, gegebenenfalls durch Halogen, OH, $NH_2$ substituiertes Alkyl($C_1$-$C_4$)thio, gegebenenfalls durch Halogen, OH, $NH_2$ substituiertes Alkyl($C_1$-$C_4$)thionyl, gegebenenfalls durch Halogen, OH, $NH_2$ substituiertes Alkyl($C_1$-$C_4$)sulfonyl, OH, SH, $NH_2$ stehen

und wobei $R_c$ und $R_d$ zusammen für eine chemische Bindung stehen,

B) $R_a$ und $R_b$ stehen zusammen für den Rest

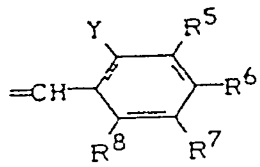

stehen,
wobei Y, $R^5$, $R^6$, $R^7$ und $R^8$ die unter A) angegebene Bedeutung besitzen und wobei $R_c$ und $R_d$ jeweils für Wasserstoff stehen,
C) $R_a$, $R_b$, $R_d$ und $R_d$ stehen jeweils für Wasserstoff,
D) $R_a$ steht für Wasserstoff und $R_b$ steht für den Rest

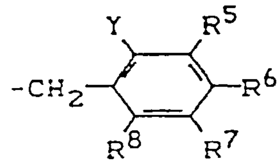

wobei Y, $R^5$, $R^6$, $R^7$ und $R^8$ die unter A) angegebene Bedeutung besitzen und $R_c$ und $R_d$ für Wasserstoff stehen, eine stark ausgeprägte Wirksamkeit gegen Spinnentiere besitzen.

Somit ergeben sich die folgenden untereinander im Zusammenhang stehenden chemischen Strukturen $I_A$, $I_B$, $I_C$ und $I_D$ :

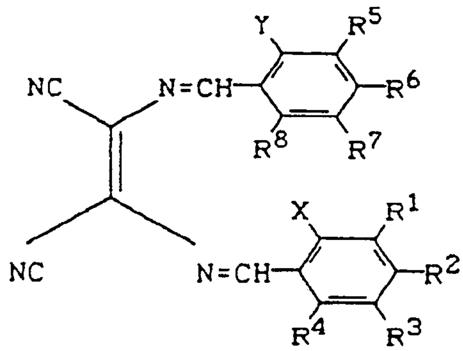

($I_A$)

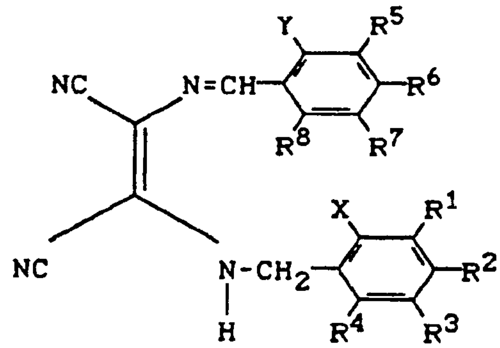

($I_B$)

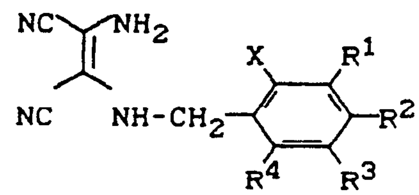

$(I_C)$

und

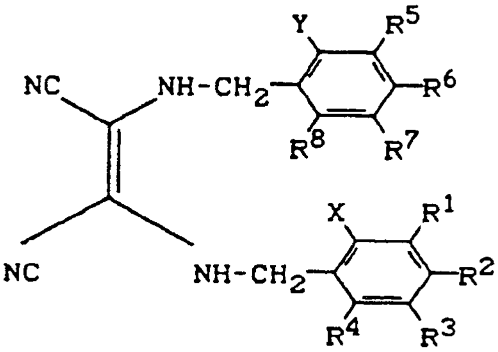

$(I_D)$

Bei den Verbindungen der Formeln $(I_B)$, $(I_C)$ und $(I_D)$ handelt es sich um neue Stoffe. Allgemein ist zu sagen, daß die Verbindungen der allgemeinen Formel (I) an sich sowohl in der cis-Form

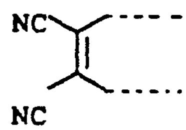 als auch in der stereoisomeren trans-Form

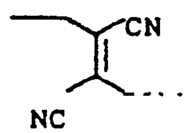 vorliegen können.

Vorzugsweise liegen die Verbindungen der allgemeinen Formel (I) in der cis-Form vor. Überraschenderweise zeigen die Verbindungen der allgemeinen Formel (I) eine stark ausgeprägte Wirksamkeit gegenüber Spinnentieren. Eine derartige Wirkung ähnlich oder gleich strukturierter Verbindungen des Standes der Technik ist bisher nicht bekannt geworden.

Man erhält die Bisanile der Formel $(I_A)$

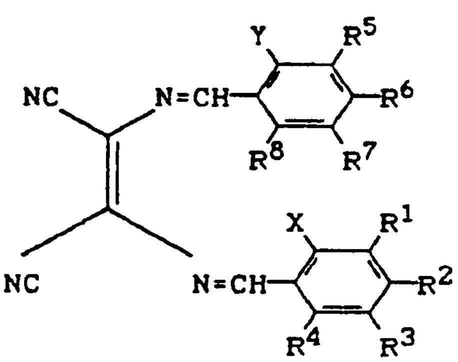

$(I_A)$

in welcher die Reste $R^1$ bis $R^8$ sowie X und Y die oben angegebene Bedeutung besitzen, indem man 2,3-Diaminomaleinsäurenitril ("DAMN") der Formel (II)

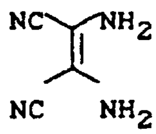

(II)

in äquimolaren Mengen in einem Verdünnungsmittel mit einem Aldehyd der Formel (III)

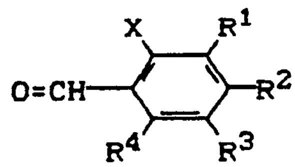

(III)

in welcher

X, $R^1$, $R^2$, $R^3$ und $R^4$ die oben angegebene Bedeutung besitzen, zum Azomethin der Formel

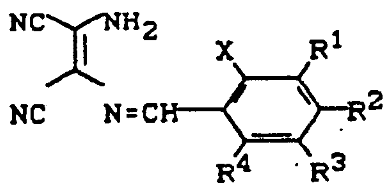

(IV)

umsetzt und dieses dann unter Zusatz eines Katalysators mit molarer Menge eines weiteren Aldehyds der Formel (V)

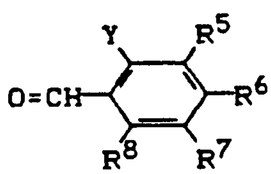

(V)

in welcher Y, $R^5$, $R^6$, $R^7$, $R^8$ die obengenannte Bedeutung haben, umsetzt.

Weiterhin erhält man die symmetrischen Bisanile der Formel (VI)

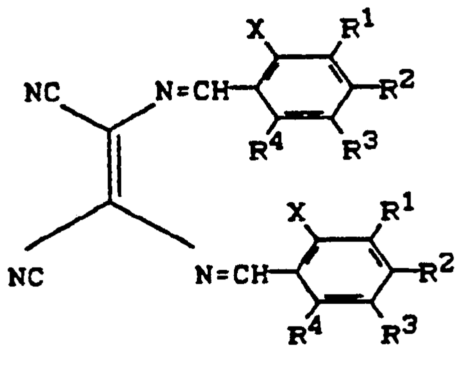

(VI)

d.h. Verbindungen der Formel $I_A$ für welche gilt : ($X = Y$, $R^1 = R^5$, $R^2 = R^6$, $R^3 = R^7$, $R^4 = R^8$), indem man 1 Mol 2,3-Diaminomaleinsäurenitril (DAMN) der Formel (II)

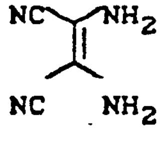

(II)

in einem Verdünnungsmittel unter Zusatz eines Katalysators mit 2 Mol eines Aldehyds der Formel (III)

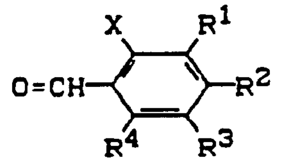

(III)

in welcher X, $R^1$, $R^2$, $R^3$ und $R^4$ die obengenannte Bedeutung haben, umsetzt.

Die Verbindungen der allgemeinen Formel ($I_A$) können in den stereoisomeren cis- und/oder trans-Formen vorliegen. Vorzugsweise liegen die Verbindungen jedoch in der cis-Form vor.

Die akarizid wirksamen Bisanile sind durch die Formel ($I_A$) allgemein definiert.

In der Formel ($I_A$) stehen X und Y, welche gleich oder verschieden sein können, vorzugsweise für Fluor, Chlor, Brom, Iod, $CF_3$ oder CN.

Bevorzugt sind Verbindungen der Formel ($I_A$), in welcher

X und $R^4$ sowie Y und $R^8$ gleich oder verschieden sein können und für Wasserstoff, Fluor, Chlor, Brom, Iod, $CF_3$ oder CN stehen,

$R^1$, $R^2$ und $R^3$ sowie $R^5$, $R^6$ und $R^7$ gleich oder verschieden sein können und für Wasserstoff, Alkyl($C_1$-$C_4$), Halogenalkyl($C_1$-$C_4$), Alkoxy($C_1$-$C_4$), Halogenalkoxy($C_1$-$C_4$), Halogen, CN, $NO_2$, Dialkyl($C_1$-$C_4$)amino, Alkoxy($C_1$-$C_4$)carbonyl, Alkyl($C_1$-$C_4$)thio, Alkyl($C_1$-$C_4$)thionyl, Dihalogenalkyl($C_1$-$C_4$)amino, Alkyl($C_1$-$C_4$)sulfonyl, OH, SH, $NH_2$ stehen.

Besonders bevorzugt sind Verbindungen der allgemeinen Formel ($I_{A'}$)

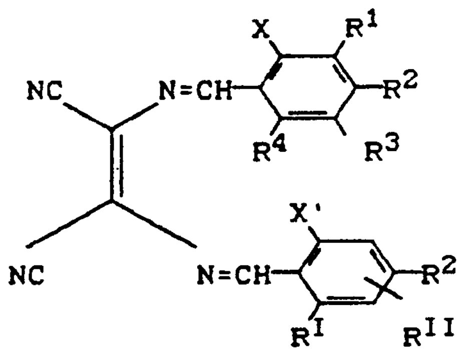

($I_{A'}$)

in der

X, $R^1$, $R^2$, $R^3$ und $R^4$ die oben angegebene Bedeutung haben und

X' und $R^I$ gleich oder verschieden sein können und für Fluor, Chlor, Brom, Iod oder $CF_3$ stehen

und

$R^{II}$ für Wasserstoff, Chlor, Brom, Iod, Cyan, Nitro, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Pentafluorethyl, Trifluorethyl, Trifluormethylsulfonyl, Trifluormethoxy, Trichlormethoxy, Pentafluorethoxy, Dimethylamino, Diethylamino, Di-β-chlorethylamino, Di-β-hydroxyethylamino steht.

Ganz besonders bevorzugt sind Verbindungen der Formel ($I_{A''}$)

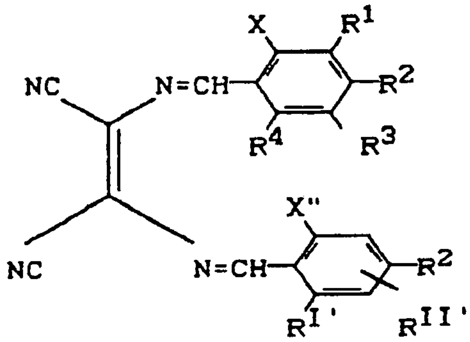

$(I_{A''})$

in der

X, $R^1$, $R^2$, $R^3$ und $R^4$ die oben angegebene Bedeutung haben und

X'' und $R^{I}$ gleich oder verschieden sein können und für Chlor oder Brom stehen

und

$R^{II'}$ für Chlor, Brom, Iod, Wasserstoff, Cyan, Nitro, Methyl, Ethyl, n- und i-Propyl, Methoxy, Ethoxy, Methoxycarbonyl, Ethoxycarbonyl, Trifluormethyl, Trichlormethyl, Dimethylamino, Diethylamino, Trifluormethylsulfon steht.

Außerordentlich bevorzugt sind Verbindungen der Formel $(I_{A'''})$

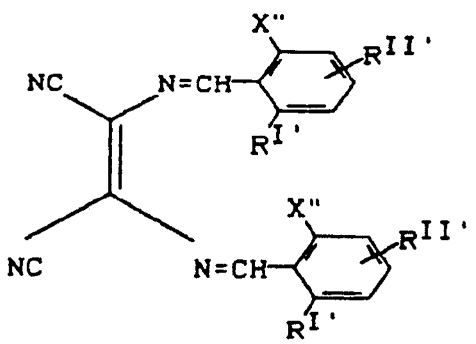

$(I_{A'''})$

in der

X'' und $R^{I'}$ gleich oder verschieden sein können und für Chlor oder Brom stehen

und

$R^{II'}$ für Chlor, Brom, Iod, Wasserstoff, Cyan, Nitro, Methyl, Ethyl, n- und i-Propyl, Methoxy, Ethoxy, Methoxycarbonyl, Ethoxycarbonyl, Trifluormethyl, Trichlormethyl, Dimethylamino, Diethylamino, Trifluormethylsulfon steht.

Von ganz besonderem Interesse sind Verbindungen der Formel $(I_{A''''})$

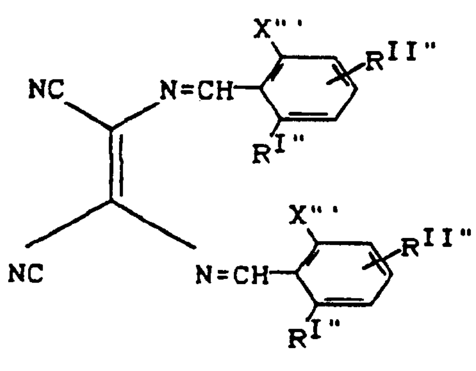

$(I_{A''''})$

in der

X''' und $R^{I''}$ gleich oder verschieden sein können und für Chlor oder Brom stehen

und

$R^{II''}$ für Wasserstoff, Chlor, Brom, Methyl, Cyano, Trichlormethyl, Trifluormethyl, Methoxy, Trifluormethoxy, Trichlormethoxy oder Trifluormethylsulfon steht.

Außerordentlich bevorzugt sind Verbindungen der Formel $(I_{A''''})$

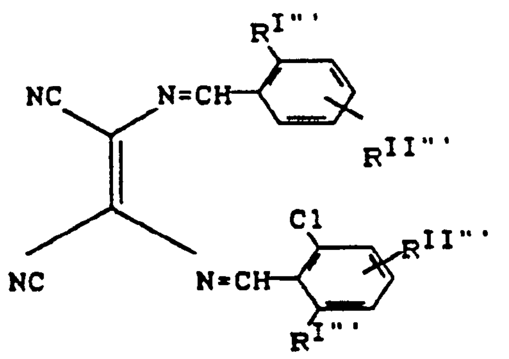

$(I_{A''''})$

in der

$R^{I''}$ für Chlor oder Brom steht und

$R^{II''}$ für Wasserstoff, Chlor, Methyl, Trichlormethyl, Methoxy, Trichlormethoxy oder Trifluormethoxy steht.

Verwendet man beispielsweise 2,3-Diaminomaleinsäurenitril (DAMN) und 2,6-Dichlorbenzaldehyd als Ausgangsprodukte, so kann das entsprechende Bisanil wie folgt dargestellt werden :

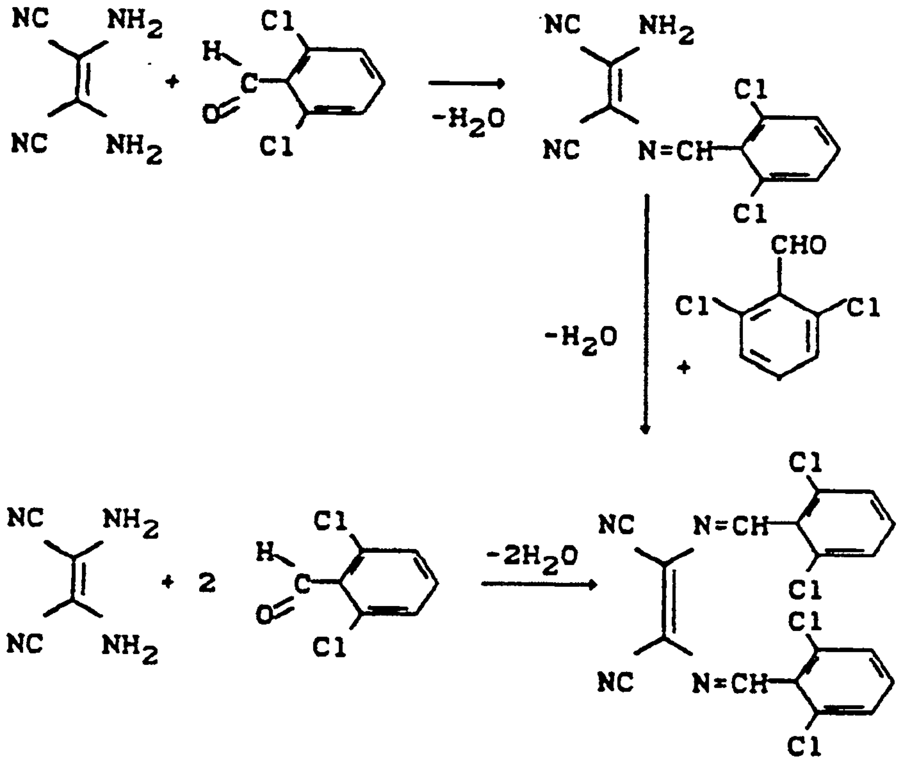


Die zur Durchführung des Verfahrens zur Herstellung der Bisanile $(I_A)$ als Ausgangsstoffe benötigten Aldehyde sind durch die Formeln (III) bzw. (V) allgemein definiert. Es handelt sich im wesentlichen um bekannte Verbindungen. Siehe dazu z.B. O. Bayer, in Houben-Weyl, Bd. VII/1, S. 16-36 (1954).

Die weitere Ausgangsverbindung 2,3-Diaminomaleinsäurenitril ist ebenfalls literaturbekannt und im Handel erhältlich.

Als Verdünnungsmittel zur Durchführung des Verfahrens zur Herstellung der erfindungsgemäß verwendbaren Verbindungen der Formeln $(I_A)$ bzw. (VI) kommen vorzugsweise polare organische Lösungsmittel in Frage, wie z.B. Alkohole (insbesondere Methanol, Ethanol, Propanol (n und i)), Dimethylformamid (DMF), Dimethylsulfoxid (DMSO), Hexamethylphosphorsäuretriamid (HMPT), Acetonitril.

Die Reaktionstemperatur liegt im allgemeinen im Bereich zwischen ca. 0°C bis maximal zum Siedepunkt des jeweiligen Lösungsmittels, insbesondere wird die Reaktion bei Temperaturen von ca. 20 bis ca. 100°C durchgeführt.

Die Umsetzung wird vorzugsweise bei Normaldruck durchgeführt.

Als Katalysator für die Umsetzung der Verbindungen der Formel (IV) mit molaren Mengen des Aldehyds der Formel (V) wird vorzugsweise eingesetzt: anorganische oder organische Säuren oder Derivate. Beispielhaft seien genannt: Chlorwasserstoffsäure, Bromwasserstoffsäure, Jodwasserstoffsäure, Schwefelsäure, Bortrifluorid, Tetrafluorborsäure, Perchlorsäure, Methansulfonsäure, Trifluormethansulfonsäure, p-Toluolsulfonsäure, Phosphorsäure, Phosphorpentoxid, Ameisensäure, Essigsäure, Propionsäure, Chlorwasserstoff, Bromwasserstoff.

Hierbei wird 0,001 bis 2 Mol Katalysator pro Mol Aldehyd der Formel (V) eingesetzt, vorzugsweise 0,01 bis 1 Mol Katalysator pro Mol Aldehyd der Formel (V).

Für die direkte Umsetzung von 2,3-Diaminomaleinsäurenitril mit 2 Mol Aldehyd der Formel (III) zum Erhalt der symmetrischen Bisanile der Formel (VI) werden vorzugsweise die obengenannten Katalysatoren in den obengenannten Mengenverhältnissen eingesetzt.

Die Verbindungen der Formel (IV)

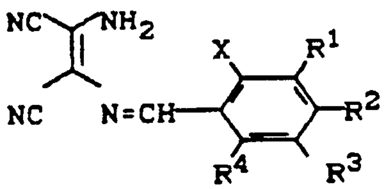

(IV)

worin X, $R^1$, $R^2$, $R^3$ und $R^4$ die oben angegebene Bedeutung besitzen,
sind Gegenstand einer älteren im Hinblick auf die vorliegende Anmeldung nicht vorveröffentlichten Patentanmeldung.

Die Verbindungen der Formel (IV) sind dadurch zugänglich, daß man das bekannte 2,3-Diaminomaleinsäurenitril der Formel

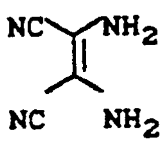

(II)

in äquimolarer Menge in einem geeigneten (vorzugsweise polaren organischen) Lösungsmittel mit einem Aldehyd der Formel

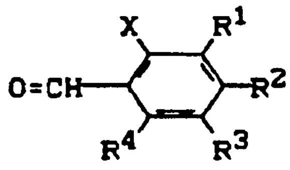

(III)

in welcher X, $R^1$, $R^2$, $R^3$ und $R^4$ die oben angegebene Bedeutung besitzen, umsetzt und die Reaktionsprodukte nach an sich bekannten Methoden aufarbeitet.

Im einzelnen seien außer den bei den Herstellungsbeispielen genannten Verbindungen die folgenden Bisanile der Formel ($I_A$) genannt:

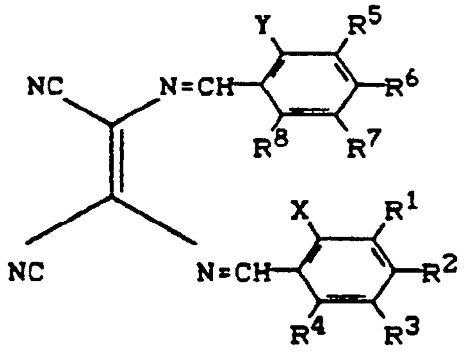

$(I_A)$

| X | $R^1$ | $R^2$ | $R^3$ | $R^4$ | Y | $R^5$ | $R^6$ | $R^7$ | $R^8$ |
|---|---|---|---|---|---|---|---|---|---|
| H | H | H | H | H | Cl | H | H | H | H |
| H | H | H | H | H | Cl | H | Cl | H | H |
| H | H | H | H | H | Cl | H | Cl | H | Cl |
| H | H | H | H | H | Cl | H | H | H | F |
| H | H | H | H | H | H | $CF_3$ | H | H | H |
| H | H | H | H | H | $CF_3$ | H | H | H | $CF_3$ |
| H | H | H | H | H | Cl | $OCF_3$ | Cl | H | Cl |
| H | H | H | H | H | F | F | F | F | F |
| H | H | H | H | H | F | F | OH | F | F |
| H | H | H | H | H | $CF_3$ | H | F | H | $CF_3$ |
| H | H | H | H | H | Cl | H | F | H | Cl |
| H | H | H | H | H | Br | H | H | H | Cl |
| H | H | H | H | H | Cl | H | $CF_3$ | H | H |
| H | H | H | H | H | Cl | Cl | $CF_3$ | H | Cl |
| H | H | H | H | H | Cl | H | $COOCH_3$ | H | Cl |
| H | H | H | H | H | Cl | H | $OCH_3$ | H | Cl |
| H | H | H | H | H | Cl | H | $SCF_3$ | H | Cl |
| H | H | H | H | H | Cl | H | $SO_2CF_3$ | H | Cl |

| X | $R^1$ | $R^2$ | $R^3$ | $R^4$ | Y | $R^5$ | $R^6$ | $R^7$ | $R^8$ |
|---|---|---|---|---|---|---|---|---|---|
| H | H | H | H | H | Cl | H | $CH_3$ | H | H |
| H | H | H | H | H | Br | H | H | H | F |
| H | H | H | H | H | H | $OCH_3$ | OH | H | H |
| H | H | H | H | H | H | H | Cl | $NO_2$ | H |
| H | H | H | H | H | CN | H | H | H | Cl |
| H | H | H | H | H | H | H | CN | H | H |
| H | H | H | H | H | H | Br | OH | $OCH_3$ | H |
| Cl | H | H | H | H | Cl | H | H | H | Cl |
| Cl | H | H | H | H | Cl | H | Cl | H | H |
| Cl | H | H | H | H | Cl | H | $CH_3$ | H | H |
| Cl | H | H | H | H | CN | H | H | H | H |
| Cl | H | H | H | H | Br | H | Br | H | Br |
| Cl | H | H | H | H | Br | H | H | H | Cl |
| Cl | H | H | H | H | Cl | H | F | H | Cl |
| Cl | H | H | H | H | $CF_3$ | H | H | H | $CF_3$ |
| Cl | H | H | H | H | H | $CF_3$ | H | H | H |
| Cl | H | H | H | H | $CF_3$ | H | F | H | $CF_3$ |
| Cl | H | H | H | H | Cl | H | H | H | F |

| X | $R^1$ | $R^2$ | $R^3$ | $R^4$ | Y | $R^5$ | $R^6$ | $R^7$ | $R^8$ |
|---|---|---|---|---|---|---|---|---|---|
| Cl | H | H | H | H | Cl | $OCF_3$ | Cl | H | Cl |
| Cl | H | H | H | H | H | Cl | H | Cl | OH |
| H | Cl | H | H | H | Cl | H | H | H | Cl |
| H | Cl | H | H | H | Cl | H | Cl | H | H |
| H | Cl | H | H | H | Cl | H | $CH_3$ | H | H |
| H | Cl | H | H | H | CN | H | H | H | H |
| H | Cl | H | H | H | Br | H | H | H | Br |
| H | Cl | H | H | H | Br | H | Br | H | Br |
| H | Cl | H | H | H | Br | H | H | H | Cl |
| H | Cl | H | H | H | Cl | H | H | H | F |
| H | Cl | H | H | H | Cl | H | F | H | Cl |
| H | Cl | H | H | H | $CF_3$ | H | H | H | $CF_3$ |
| H | Cl | H | H | H | H | $CF_3$ | H | H | H |
| H | Cl | H | H | H | Cl | H | Cl | $OCF_3$ | Cl |
| H | Cl | H | H | H | H | Cl | H | Cl | OH |
| H | H | Cl | H | H | Cl | H | H | H | Cl |
| H | H | Cl | H | H | Cl | H | Cl | H | H |
| H | H | Cl | H | H | Cl | H | Cl | H | H |
| H | H | Cl | H | H | Cl | H | $CH_3$ | H | H |

| X | $R^1$ | $R^2$ | $R^3$ | $R^4$ | Y | $R^5$ | $R^6$ | $R^7$ | $R^8$ |
|---|---|---|---|---|---|---|---|---|---|
| H | H | Cl | H | H | CN | H | H | H | H |
| H | H | Cl | H | H | Br | H | H | H | Br |
| H | H | Cl | H | H | Br | H | Br | H | Br |
| H | H | Cl | H | H | Br | H | H | H | Cl |
| H | H | Cl | H | H | Cl | H | H | H | F |
| H | H | Cl | H | H | Cl | H | F | H | Cl |
| H | H | Cl | H | H | $CF_3$ | H | H | H | $CF_3$ |
| H | H | Cl | H | H | H | $CF_3$ | H | H | H |
| H | H | Cl | H | H | Cl | H | Cl | $OCF_3$ | Cl |
| H | H | Cl | H | H | H | Cl | H | Cl | OH |
| Cl | H | H | H | Cl | Cl | H | H | H | Cl |
| Cl | H | H | H | Cl | Cl | H | Cl | H | H |
| Cl | H | H | H | Cl | Cl | H | $CH_3$ | H | H |
| Cl | H | H | H | Cl | CN | H | H | H | H |
| Cl | H | H | H | Cl | Br | H | H | H | Br |
| Cl | H | H | H | Cl | Br | H | Br | H | Br |
| Cl | H | H | H | Cl | Br | H | H | H | Cl |
| Cl | H | H | H | Cl | Cl | H | H | H | F |

| X | $R^1$ | $R^2$ | $R^3$ | $R^4$ | Y | $R^5$ | $R^6$ | $R^7$ | $R^8$ |
|---|---|---|---|---|---|---|---|---|---|
| Cl | H | H | H | Cl | Cl | H | F | H | Cl |
| Cl | H | H | H | Cl | $CF_3$ | H | H | H | $CF_3$ |
| Cl | H | H | H | Cl | H | $CF_3$ | H | H | H |
| Cl | H | H | H | Cl | Cl | H | Cl | $OCF_3$ | Cl |
| Cl | H | H | H | Cl | H | Cl | H | Cl | OH |
| Cl | H | Cl | H | H | Cl | H | H | H | Cl |
| Cl | H | Cl | H | H | Cl | H | Cl | H | H |
| Cl | H | Cl | H | H | Cl | H | $CH_3$ | H | H |
| Cl | H | Cl | H | H | CN | H | H | H | H |
| Cl | H | Cl | H | H | Br | H | H | H | Br |
| Cl | H | Cl | H | H | Br | H | Br | H | Br |
| Cl | H | Cl | H | H | Br | H | H | H | Cl |
| Cl | H | Cl | H | H | Cl | H | H | H | F |
| Cl | H | Cl | H | H | Cl | H | F | H | Cl |
| Cl | H | Cl | H | H | $CF_3$ | H | H | H | $CF_3$ |
| Cl | H | Cl | H | H | H | $CF_3$ | H | H | H |
| Cl | H | Cl | H | H | Cl | H | Cl | $OCF_3$ | Cl |
| Cl | H | Cl | H | H | H | Cl | H | Cl | OH |

| X | $R^1$ | $R^2$ | $R^3$ | $R^4$ | Y | $R^5$ | $R^6$ | $R^7$ | $R^8$ |
|---|---|---|---|---|---|---|---|---|---|
| H | $CF_3$ | H | H | H | Cl | H | H | H | Cl |
| H | $CF_3$ | H | H | H | Cl | H | Cl | H | H |
| H | $CF_3$ | H | H | H | Cl | H | $CH_3$ | H | H |
| H | $CF_3$ | H | H | H | CN | H | H | H | H |
| H | $CF_3$ | H | H | H | Br | H | H | H | Br |
| H | $CF_3$ | H | H | H | Br | H | Br | H | Br |
| H | $CF_3$ | H | H | H | Br | H | H | H | Cl |
| H | $CF_3$ | H | H | H | Cl | H | H | H | F |
| H | $CF_3$ | H | H | H | Cl | H | F | H | Cl |
| H | $CF_3$ | H | H | H | $CF_3$ | H | H | H | $CF_3$ |
| H | $CF_3$ | H | H | H | H | $CF_3$ | H | H | H |
| H | $CF_3$ | H | H | H | Cl | H | Cl | $OCF_3$ | Cl |
| H | $CF_3$ | H | H | H | H | Cl | H | Cl | OH |

| X | $R^1$ | $R^2$ | $R^3$ | $R^4$ | Y | $R^5$ | $R^6$ | $R^7$ | $R^8$ |
|---|---|---|---|---|---|---|---|---|---|
| Cl | H | H | H | F | Cl | H | H | H | Cl |
| Cl | H | H | H | F | Cl | H | Cl | H | H |
| Cl | H | H | H | F | Cl | H | $CH_3$ | H | H |
| Cl | H | H | H | F | CN | H | H | H | H |
| Cl | H | H | H | F | Br | H | H | H | Br |
| Cl | H | H | H | F | Br | H | Br | H | Br |
| Cl | H | H | H | F | Br | H | H | H | Cl |
| Cl | H | H | H | F | Cl | H | H | H | F |
| Cl | H | H | H | F | Cl | H | F | H | Cl |
| Cl | H | H | H | F | $CF_3$ | H | H | H | $CF_3$ |
| Cl | H | H | H | F | H | $CF_3$ | H | H | H |
| Cl | H | H | H | F | Cl | H | Cl | $OCF_3$ | Cl |
| Cl | H | H | H | F | H | Cl | H | Cl | OH |

| X | $R^1$ | $R^2$ | $R^3$ | $R^4$ | Y | $R^5$ | $R^6$ | $R^7$ | $R^8$ |
|---|---|---|---|---|---|---|---|---|---|
| $CF_3$ | H | H | H | $CF_3$ | Cl | H | H | H | H |
| $CF_3$ | H | H | H | $CF_3$ | H | Cl | Cl | H | H |
| $CF_3$ | H | H | H | $CF_3$ | H | $CF_3$ | H | H | H |
| $CF_3$ | H | H | H | $CF_3$ | H | Cl | H | Cl | OH |
| $CF_3$ | H | H | H | $CF_3$ | Cl | H | Cl | H | H |
| $CF_3$ | H | H | H | $CF_3$ | Cl | H | H | H | Cl |
| Cl | H | Cl | $OCF_3$ | Cl | Cl | H | H | H | H |
| Cl | H | Cl | $OCF_3$ | Cl | Cl | H | H | H | Cl |
| Cl | H | Cl | $OCF_3$ | Cl | Cl | H | Cl | H | H |
| Cl | H | Cl | $OCF_3$ | Cl | H | Cl | Cl | H | H |
| Cl | H | Cl | $OCF_3$ | Cl | H | $CF_3$ | H | H | H |
| Cl | H | Cl | $OCF_3$ | Cl | H | $OCH_3$ | OH | Br | H |
| H | Cl | Cl | H | H | Cl | H | H | H | Cl |

| X | $R^1$ | $R^2$ | $R^3$ | $R^4$ | Y | $R^5$ | $R^6$ | $R^7$ | $R^8$ |
|---|---|---|---|---|---|---|---|---|---|
| Cl | H | $CH_3$ | H | H | Cl | H | H | H | H |
| Cl | H | $CH_3$ | H | H | H | $CF_3$ | H | H | H |
| Cl | H | $CH_3$ | H | H | Cl | H | H | H | Cl |
| Cl | H | $CH_3$ | H | H | Cl | H | Cl | H | H |
| Cl | H | $CH_3$ | H | H | H | Cl | H | Cl | OH |
| Cl | H | $CF_3$ | H | Cl | Cl | H | Cl | H | H |
| Cl | H | $CF_3$ | H | Cl | H | $CF_3$ | H | H | H |
| Cl | H | $CF_3$ | H | Cl | Cl | H | H | H | Cl |
| Cl | H | $CF_3$ | H | Cl | H | Cl | H | Cl | OH |
| Cl | H | $CF_3$ | H | Cl | H | $NO_2$ | H | H | H |
| Cl | $CF_3$ | Cl | H | H | $CF_3$ | H | $CH_3$ | H | $CH_3$ |
| Cl | $CF_3$ | Cl | H | H | Cl | H | $CH_3$ | H | H |
| H | H | $N(CH_3)_2$ | H | H | Cl | H | H | H | Cl |

Man erhält die neuen Benzyl-benzyliden-Derivate des 2,3-Diaminomaleinsäurenitrils der Formel ($I_B$)

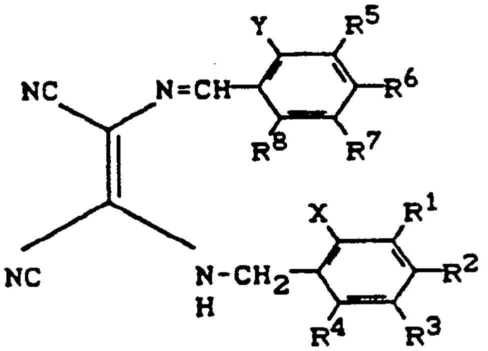

($I_B$)

in welcher X, Y, $R^1$, $R^2$, $R^3$, $R^4$, $R^5$, $R^6$, $R^7$ und $R^8$ die unter Formel ($I_A$) angegebene Bedeutung haben, wenn man Benzylverbindungen der allgemeinen Formel

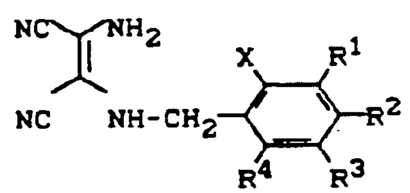

($I_C$)

in äquimolarer Menge in einem geeigneten Verdünnungsmittel mit einem Aldehyd der Formel (V)

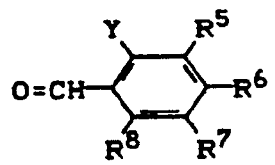

(V)

in welcher Y, $R^5$, $R^6$, $R^7$, $R^8$ die oben angegebene Bedeutung besitzen, umsetzt.

Überraschenderweise zeigen die Verbindungen der Formel ($I_B$) eine akarizide Wirksamkeit. Eine derartige Wirkung ist von der Stoffklasse der Benzyl-benzyliden-Verbindungen des Diaminomaleinsäurenitrils bisher noch nicht bekannt geworden. Die erfindungsgemäßen Verbindungen stellen somit eine Bereicherung des Standes der Technik dar.

Die Verbindungen der allgemeinen Formel ($I_B$) können in den stereoisomeren cis- und/oder trans-Formen vorliegen, vorzugsweise liegen die Verbindungen jedoch in der cis-Form vor.

In der Formel ($I_B$) stehen X bzw. Y vorzugsweise für Wasserstoff, Fluor, Chlor, Brom, Iod, $CF_3$ oder CN.

Bevorzugt sind Verbindungen der Formel ($I_B$)

in welcher

X und $R^4$ sowie Y und $R^8$ gleich oder verschieden sein können und für Wasserstoff, Fluor, Chlor, Brom, Iod, $CF_3$ oder CN stehen,

$R^1$, $R^2$ und $R^3$ sowie $R^5$, $R^6$ und $R^7$ gleich oder verschieden sein können und für Wasserstoff, Alkyl($C_1$-$C_4$), Halogenalkyl($C_1$-$C_4$), Alkoxy($C_1$-$C_4$), Halogenalkoxy($C_1$-$C_4$), Halogen, CN, $NO_2$, Dialkyl($C_1$-$C_4$)amino, Alkoxy($C_1$-$C_4$)carbonyl, Alkyl($C_1$-$C_4$)thio, Alkyl($C_1$-$C_4$)thionyl, Dihalogenalkyl($C_1$-$C_4$)amino, Alkyl($C_1$-$C_4$)sulfonyl, OH, SH, $NH_2$ stehen.

Besonders bevorzugt sind Verbindungen der allgemeinen Formel ($I_{B'}$)

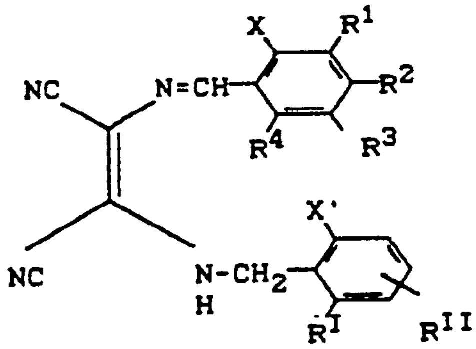

$(I_{B'})$

in der X, $R^1$, $R^2$, $R^3$ und $R^4$ die oben agegebene Bedeutung haben und

X' und $R^I$ gleich oder verschieden sein können und für Wasserstoff, Fluor, Chlor, Brom, Iod oder $CF_3$ stehen und

$R^{II}$ für Wasserstoff, Chlor, Brom, Iod, Cyan, Nitro, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Pentafluorethyl, Trifluorethyl, Trifluormethylsulfon, Trifluormethoxy, Trichlormethoxy, Pentafluorethoxy, Dimethylamino, Diethylamino, Di-β-chlorethylamino, Di-β-hydroxyethylamino steht.

Ganz besonders bevorzugt sind Verbindungen der Formel $(I_{B''})$

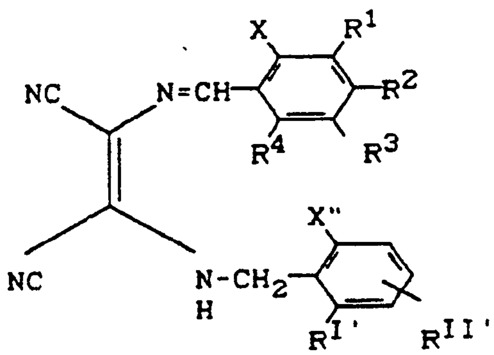

$(I_{B''})$

in der X, $R^1$, $R^2$, $R^3$ und $R^4$ die oben angegebene Bedeutung haben und

X'' und $R^{I'}$ gleich oder verschieden sein können und für Wasserstoff, Chlor oder Brom stehen und

$R^{II'}$ für Chlor, Brom, Iod, Wasserstoff, Cyan, Nitro, Methyl, Ethyl, n- und i-Propyl, Methoxy, Ethoxy, Methoxycarbonyl, Ethoxycarbonyl, Trifluormethyl, Trichlormethyl, Dimethylamino, Diethylamino, Trifluormethylsulfon steht.

Ganz besonders bevorzugt sind weiterhin Verbindungen der Formel $(I_{B'''})$

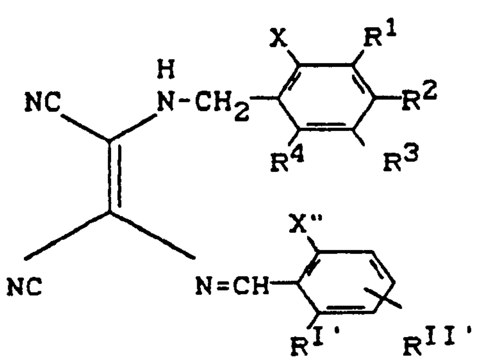

$(I_{B'''})$

in der X, $R^1$, $R^2$, $R^3$ und $R^4$ die oben angegebene Bedeutung haben und

X'' und $R^{I'}$ gleich oder verschieden sein können und für Wasserstoff, Chlor oder Brom stehen

und

$R^{II'}$ für Chlor, Brom, Iod, Wasserstoff, Cyan, Nitro, Methyl, Ethyl, n- und i-Propyl, Methoxy, Ethoxy, Methoxycarbonyl, Ethoxycarbonyl, Trifluormethyl, Trichlormethyl, Dimethylamino, Diethylamino, Trifluormethylsulfon steht.

Außerordentlich bevorzugt sind Verbindungen der Formel ($I_{B''''}$)

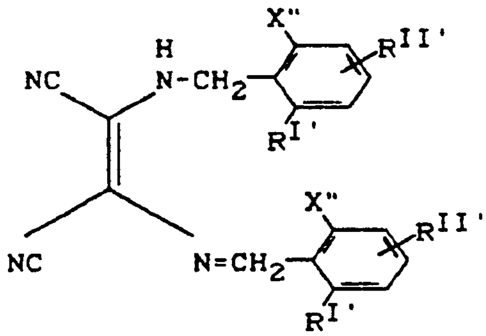

($I_{B''''}$)

in der

X'' und $R^{I'}$ gleich oder verschieden sein können und für Wasserstoff, Chlor oder Brom stehen

und

$R^{II'}$ für Chlor, Brom, Iod, Wasserstoff, Cyan, Nitro, Methyl, Ethyl, n- und i-Propyl, Methoxy, Ethoxy, Methoxycarbonyl, Ethoxycarbonyl, Trifluormethyl, Trichlormethyl, Dimethylamino, Diethylamino, Trifluormethylsulfon steht.

Extrem bevorzugt sind Verbindungen der Formel ($I_{B'''''}$)

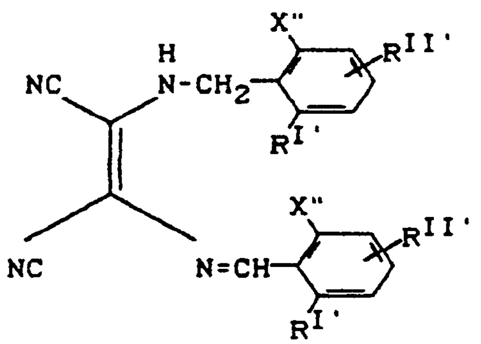

($I_{B'''''}$)

in der

X'' und $R^{I'}$ gleich oder verschieden sein können und für Wasserstoff, Chlor oder Brom stehen

und

$R^{II'}$ für Chlor, Brom, Iod, Wasserstoff, Cyan, Nitro, Methyl, Ethyl, n- und i-Propyl, Methoxy, Ethoxy, Methoxycarbonyl, Ethoxycarbonyl, Trifluormethyl, Trichlormethyl, Dimethylamino, Diethylamino, Trifluormethylsulfon steht.

Von ganz besonderem Interesse sind Verbindungen der Formel ($I_BVI$)

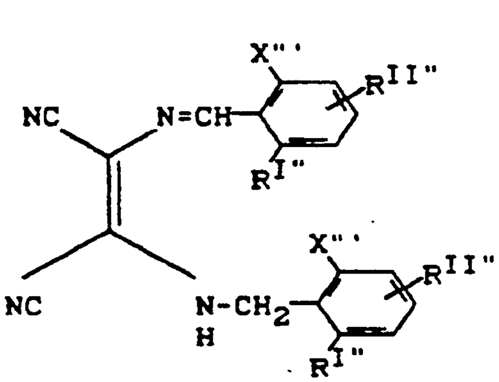

($I_BVI$)

in der

$X'''$ und $R^{I''}$ gleich oder verschieden sein können und für Wasserstoff, Chlor oder Brom stehen

und

$R^{II''}$ für Wasserstoff, Chlor, Brom, Methyl, Cyano, Trifluormethyl, Methoxy, Trifluormethoxy, Trichlormethoxy oder Trifluormethylsulfon steht.

Außerordentlich bevorzugt sind Verbindungen der allgemeinen Formel ($I_B$VII)

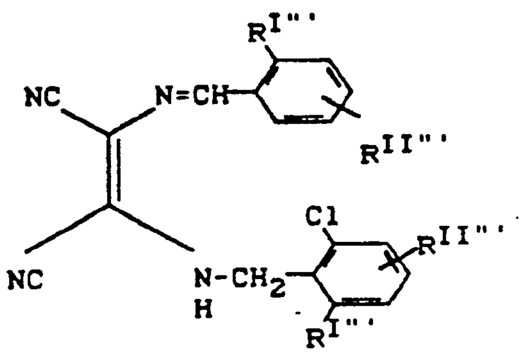

($I_B$VII)

in der

$R^{I'''}$ für Chlor oder Brom steht und

$R^{II'''}$ für Wasserstoff, Chlor, Methyl, Trichlormethyl, Methoxy, Trichlormethoxy oder Trifluormethoxy steht.

Von ganz besonderem Interesse sind Verbindungen der Formel ($I_B$VIII)

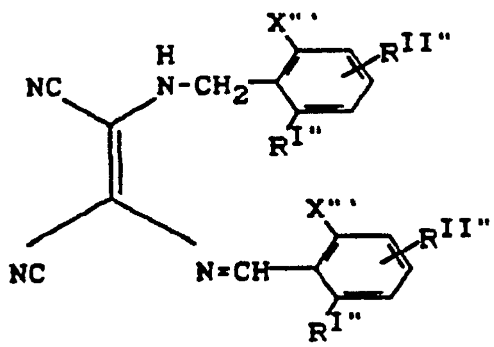

($I_B$VIII)

in der

$X'''$ und $R^{I''}$ gleich oder verschieden sein können und für Wasserstoff, Chlor oder Brom stehen

und

$R^{II''}$ für Wasserstoff, Chlor, Brom, Methyl, Cyano, Trifluormethyl, Methoxy, Trifluormethoxy, Trichlormethoxy oder Trifluormethylsulfon steht.

Außerordentlich bevorzugt sind Verbindungen der allgemeinen Formel ($I_B$IX)

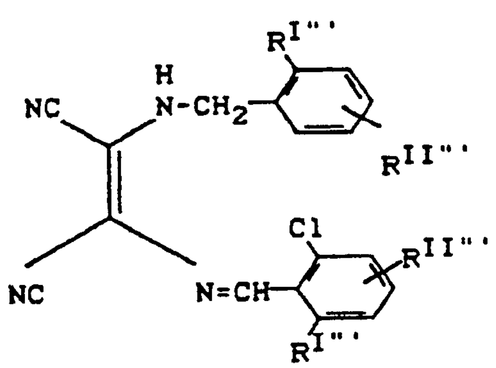

($I_B$IX)

in der

$R^{I''}$ für Chlor oder Brom steht und

$R^{II''}$ für Wasserstoff, Chlor, Methyl, Trichlormethyl, Methoxy, Trichlormethoxy oder Trifluormethoxy steht.

Verwendet man beispielsweise die 2,4-Dichlorbenzyl-Verbindung des 2,3-Diaminomaleinsäurenitrils und den 2,6-Dichlorbenzaldehyd als Ausgangsprodukte, so kann das Herstellungsverfahren zum Erhalt der Verbindungen $I_B$ wie folgt dargestellt werden :

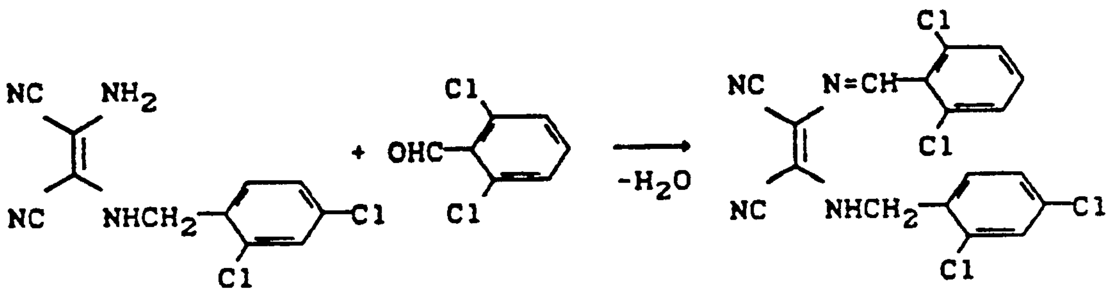


Die zur Durchführung des erfindungsgemäßen Verfahrens als Ausgangsstoffe benötigten Aldehyde sind durch die Formel (V) allgemein definiert. Es handelt sich im wesentlichen um bekannte Verbindungen. Siehe dazu z.B. O. Bayer, in Houben-Weyl, Bd. VII/1, S. 16-36 (1954).

Als Verdünnungsmittel zur Durchführung des erfindungsgemäßen Verfahrens kommen vorzugsweise polare organische Lösungsmittel in Frage, wie z.B. Alkohole (insbesondere Methanol, Ethanol, Propanol (n und i)), Dimethylformamid (DMF), Dimethylsulfoxid (DMSO), Hexamethylphosphorsäuretriamid (HMPT), Acetonitril.

Die Reaktionstemperatur liegt im allgemeinen im Bereich zwischen ca. 0°C bis maximal zum Siedepunkt des jeweiligen Lösungsmittels, insbesondere wird die Reaktion bei Temperaturen von ca. 20 bis ca. 100°C durchgeführt.

Die Umsetzung wird vorzugsweise bei Normaldruck durchgeführt.

Zur Durchführung des erfindungsgemäßen Verfahrens setzt man bevorzugt äquimolare Mengen der Reaktionspartner ($I_C$) und (V) miteinander um. Es kann aber auch ein leichter Überschuß eines der beiden Reaktionspartner angewendet werden.

In einer bevorzugten Ausführungsform gibt man bei Raumtemperatur die beiden Reaktionspartner im Verdünnungsmittel zusammen und erhitzt anschließend zum Rückfluß.

Die Aufarbeitung erfolgt nach üblichen Methoden, nach dem Abkühlen wird das Reaktionsprodukt vorzugsweise abgesaugt und nach an sich bekannten Methoden aufgearbeitet.

Im einzelnen seien außer den bei den Herstellungsbeispielen genannten Verbindungen die folgenden Benzylbenzyliden-Verbindungen der Formel ($I_B$) genannt :

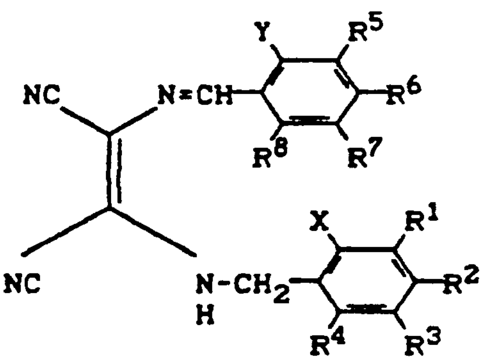

($I_B$)

| X | $R^1$ | $R^2$ | $R^3$ | $R^4$ | Y | $R^5$ | $R^6$ | $R^7$ | $R^8$ |
|---|---|---|---|---|---|---|---|---|---|
| H | H | H | H | H | Cl | H | H | H | H |
| H | H | H | H | H | Cl | H | Cl | H | H |
| H | H | H | H | H | Cl | H | Cl | H | Cl |
| H | H | H | H | H | Cl | H | H | H | F |
| H | H | H | H | H | H | $CF_3$ | H | H | H |
| H | H | H | H | H | $CF_3$ | H | H | H | $CF_3$ |
| H | H | H | H | H | Cl | $OCF_3$ | Cl | H | Cl |
| H | H | H | H | H | F | F | F | F | F |
| H | H | H | H | H | F | F | OH | F | F |
| H | H | H | H | H | $CF_3$ | H | F | H | $CF_3$ |
| H | H | H | H | H | Cl | H | F | H | Cl |
| H | H | H | H | H | Br | H | H | H | Cl |
| H | H | H | H | H | Cl | H | $CF_3$ | H | H |
| H | H | H | H | H | Cl | Cl | $CF_3$ | H | Cl |
| H | H | H | H | H | Cl | H | $COOCH_3$ | H | Cl |
| H | H | H | H | H | Cl | H | $OCH_3$ | H | Cl |
| H | H | H | H | H | Cl | H | $SCF_3$ | H | Cl |
| H | H | H | H | H | Cl | H | $SO_2CF_3$ | H | Cl |

| X | $R^1$ | $R^2$ | $R^3$ | $R^4$ | Y | $R^5$ | $R^6$ | $R^7$ | $R^8$ |
|---|---|---|---|---|---|---|---|---|---|
| H | H | H | H | H | Cl | H | $CH_3$ | H | H |
| H | H | H | H | H | Br | H | H | H | F |
| H | H | H | H | H | H | $OCH_3$ | OH | H | H |
| H | H | H | H | H | H | H | Cl | $NO_2$ | H |
| H | H | H | H | H | CN | H | H | H | Cl |
| H | H | H | H | H | H | H | CN | H | H |
| H | H | H | H | H | H | Br | OH | $OCH_3$ | H |
| Cl | H | H | H | H | Cl | H | H | H | Cl |
| Cl | H | H | H | H | Cl | H | Cl | H | H |
| Cl | H | H | H | H | Cl | H | $CH_3$ | H | H |
| Cl | H | H | H | H | CN | H | H | H | H |
| Cl | H | H | H | H | Br | H | Br | H | Br |
| Cl | H | H | H | H | Br | H | H | H | Cl |
| Cl | H | H | H | H | Cl | H | F | H | Cl |
| Cl | H | H | H | H | $CF_3$ | H | H | H | $CF_3$ |
| Cl | H | H | H | H | H | $CF_3$ | H | H | H |
| Cl | H | H | H | H | $CF_3$ | H | F | H | $CF_3$ |
| Cl | H | H | H | H | Cl | H | H | H | F |

| X | $R^1$ | $R^2$ | $R^3$ | $R^4$ | Y | $R^5$ | $R^6$ | $R^7$ | $R^8$ |
|---|---|---|---|---|---|---|---|---|---|
| Cl | H | H | H | H | Cl | $OCF_3$ | Cl | H | Cl |
| Cl | H | H | H | H | H | Cl | H | Cl | OH |
| H | Cl | H | H | H | Cl | H | H | H | Cl |
| H | Cl | H | H | H | Cl | H | Cl | H | H |
| H | Cl | H | H | H | Cl | H | $CH_3$ | H | H |
| H | Cl | H | H | H | CN | H | H | H | H |
| H | Cl | H | H | H | Br | H | H | H | Br |
| H | Cl | H | H | H | Br | H | Br | H | Br |
| H | Cl | H | H | H | Br | H | H | H | Cl |
| H | Cl | H | H | H | Cl | H | H | H | F |
| H | Cl | H | H | H | Cl | H | F | H | Cl |
| H | Cl | H | H | H | $CF_3$ | H | H | H | $CF_3$ |
| H | Cl | H | H | H | H | $CF_3$ | H | H | H |
| H | Cl | H | H | H | Cl | H | Cl | $OCF_3$ | Cl |
| H | Cl | H | H | H | H | Cl | H | Cl | OH |
| H | H | Cl | H | H | Cl | H | H | H | Cl |
| H | H | Cl | H | H | Cl | H | Cl | H | H |
| H | H | Cl | H | H | Cl | H | Cl | H | Cl |
| H | H | Cl | H | H | Cl | H | $CH_3$ | H | H |

| X | $R^1$ | $R^2$ | $R^3$ | $R^4$ | Y | $R^5$ | $R^6$ | $R^7$ | $R^8$ |
|---|---|---|---|---|---|---|---|---|---|
| H | H | Cl | H | H | CN | H | H | H | H |
| H | H | Cl | H | H | Br | H | H | H | Br |
| H | H | Cl | H | H | Br | H | Br | H | Br |
| H | H | Cl | H | H | Br | H | H | H | Cl |
| H | H | Cl | H | H | Cl | H | H | H | F |
| H | H | Cl | H | H | Cl | H | F | H | Cl |
| H | H | Cl | H | H | $CF_3$ | H | H | H | $CF_3$ |
| H | H | Cl | H | H | H | $CF_3$ | H | H | H |
| H | H | Cl | H | H | Cl | H | Cl | $OCF_3$ | Cl |
| H | H | Cl | H | H | H | Cl | H | Cl | OH |
| Cl | H | H | H | Cl | Cl | H | H | H | Cl |
| Cl | H | H | H | Cl | Cl | H | Cl | H | H |
| Cl | H | H | H | Cl | Cl | H | $CH_3$ | H | H |
| Cl | H | H | H | Cl | CN | H | H | H | H |
| Cl | H | H | H | Cl | Br | H | H | H | Br |
| Cl | H | H | H | Cl | Br | H | Br | H | Br |
| Cl | H | H | H | Cl | Br | H | H | H | Cl |
| Cl | H | H | H | Cl | Cl | H | H | H | F |

| X | $R^1$ | $R^2$ | $R^3$ | $R^4$ | Y | $R^5$ | $R^6$ | $R^7$ | $R^8$ |
|---|---|---|---|---|---|---|---|---|---|
| Cl | H | H | H | Cl | Cl | H | F | H | Cl |
| Cl | H | H | H | Cl | $CF_3$ | H | H | H | $CF_3$ |
| Cl | H | H | H | Cl | H | $CF_3$ | H | H | H |
| Cl | H | H | H | Cl | Cl | H | Cl | $OCF_3$ | Cl |
| Cl | H | H | H | Cl | H | Cl | H | Cl | OH |
| Cl | H | Cl | H | H | Cl | H | H | H | Cl |
| Cl | H | Cl | H | H | Cl | H | Cl | H | H |
| Cl | H | Cl | H | H | Cl | H | $CH_3$ | H | H |
| Cl | H | Cl | H | H | CN | H | H | H | H |
| Cl | H | Cl | H | H | Br | H | H | H | Br |
| Cl | H | Cl | H | H | Br | H | Br | H | Br |
| Cl | H | Cl | H | H | Br | H | H | H | Cl |
| Cl | H | Cl | H | H | Cl | H | H | H | F |
| Cl | H | Cl | H | H | Cl | H | F | H | Cl |
| Cl | H | Cl | H | H | $CF_3$ | H | H | H | $CF_3$ |
| Cl | H | Cl | H | H | H | $CF_3$ | H | H | H |
| Cl | H | Cl | H | H | Cl | H | Cl | $OCF_3$ | Cl |
| Cl | H | Cl | H | H | H | Cl | H | Cl | OH |

| X | $R^1$ | $R^2$ | $R^3$ | $R^4$ | Y | $R^5$ | $R^6$ | $R^7$ | $R^8$ |
|---|---|---|---|---|---|---|---|---|---|
| H | $CF_3$ | H | H | H | Cl | H | H | H | Cl |
| H | $CF_3$ | H | H | H | Cl | H | Cl | H | H |
| H | $CF_3$ | H | H | H | Cl | H | $CH_3$ | H | H |
| H | $CF_3$ | H | H | H | CN | H | H | H | H |
| H | $CF_3$ | H | H | H | Br | H | H | H | Br |
| H | $CF_3$ | H | H | H | Br | H | Br | H | Br |
| H | $CF_3$ | H | H | H | Br | H | H | H | Cl |
| H | $CF_3$ | H | H | H | Cl | H | H | H | F |
| H | $CF_3$ | H | H | H | Cl | H | F | H | Cl |
| H | $CF_3$ | H | H | H | $CF_3$ | H | H | H | $CF_3$ |
| H | $CF_3$ | H | H | H | H | $CF_3$ | H | H | H |
| H | $CF_3$ | H | H | H | Cl | H | Cl | $OCF_3$ | Cl |
| H | $CF_3$ | H | H | H | H | Cl | H | Cl | OH |

| X | $R^1$ | $R^2$ | $R^3$ | $R^4$ | Y | $R^5$ | $R^6$ | $R^7$ | $R^8$ |
|---|---|---|---|---|---|---|---|---|---|
| Cl | H | H | H | F | Cl | H | H | H | Cl |
| Cl | H | H | H | F | Cl | H | Cl | H | H |
| Cl | H | H | H | F | Cl | H | $CH_3$ | H | H |
| Cl | H | H | H | F | CN | H | H | H | H |
| Cl | H | H | H | F | Br | H | H | H | Br |
| Cl | H | H | H | F | Br | H | Br | H | Br |
| Cl | H | H | H | F | Br | H | H | H | Cl |
| Cl | H | H | H | F | Cl | H | H | H | F |
| Cl | H | H | H | F | Cl | H | F | H | Cl |
| Cl | H | H | H | F | $CF_3$ | H | H | H | $CF_3$ |
| Cl | H | H | H | F | H | $CF_3$ | H | H | H |
| Cl | H | H | H | F | Cl | H | Cl | $OCF_3$ | Cl |
| Cl | H | H | H | F | H | Cl | H | Cl | OH |

| X | $R^1$ | $R^2$ | $R^3$ | $R^4$ | Y | $R^5$ | $R^6$ | $R^7$ | $R^8$ |
|---|---|---|---|---|---|---|---|---|---|
| $CF_3$ | H | H | H | $CF_3$ | Cl | H | H | H | H |
| $CF_3$ | H | H | H | $CF_3$ | H | Cl | Cl | H | H |
| $CF_3$ | H | H | H | $CF_3$ | H | $CF_3$ | H | H | H |
| $CF_3$ | H | H | H | $CF_3$ | H | Cl | H | Cl | OH |
| $CF_3$ | H | H | H | $CF_3$ | Cl | H | Cl | H | H |
| $CF_3$ | H | H | H | $CF_3$ | Cl | H | H | H | Cl |
| Cl | H | Cl | $OCF_3$ | Cl | Cl | H | H | H | H |
| Cl | H | Cl | $OCF_3$ | Cl | Cl | H | H | H | Cl |
| Cl | H | Cl | $OCF_3$ | Cl | Cl | H | Cl | H | H |
| Cl | H | Cl | $OCF_3$ | Cl | H | Cl | Cl | H | H |
| Cl | H | Cl | $OCF_3$ | Cl | H | $CF_3$ | H | H | H |
| Cl | H | Cl | $OCF_3$ | Cl | H | $OCH_3$ | OH | Br | H |
| H | Cl | Cl | H | H | Cl | H | H | H | Cl |

| X | $R^1$ | $R^2$ | $R^3$ | $R^4$ | Y | $R^5$ | $R^6$ | $R^7$ | $R^8$ |
|---|---|---|---|---|---|---|---|---|---|
| Cl | H | $CH_3$ | H | H | Cl | H | H | H | H |
| Cl | H | $CH_3$ | H | H | H | $CF_3$ | H | H | H |
| Cl | H | $CH_3$ | H | H | Cl | H | H | H | Cl |
| Cl | H | $CH_3$ | H | H | Cl | H | Cl | H | H |
| Cl | H | $CH_3$ | H | H | H | Cl | H | Cl | OH |
| Cl | H | $CF_3$ | H | Cl | Cl | H | Cl | H | H |
| Cl | H | $CF_3$ | H | Cl | H | $CF_3$ | H | H | H |
| Cl | H | $CF_3$ | H | Cl | Cl | H | H | H | Cl |
| Cl | H | $CF_3$ | H | Cl | H | Cl | H | Cl | OH |
| Cl | H | $CF_3$ | H | Cl | H | $NO_2$ | H | H | H |
| Cl | $CF_3$ | Cl | H | H | $CH_3$ | H | $CH_3$ | H | $CH_3$ |
| Cl | $CF_3$ | Cl | H | H | Cl | H | $CH_3$ | H | H |
| H | H | $N(CH_3)_2$ | H | H | Cl | H | H | H | Cl |

Man erhält die neuen Benzyl-Derivate des 2,3-Diaminomaleinsäurenitrils der Formel ($I_C$)

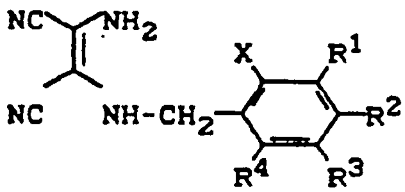

($I_C$)

in welcher X, $R^1$, $R^2$, $R^3$ und $R^4$ die unter der Formel (I) angegebene Bedeutung besitzen, wenn man Azomethine von 2,3-Diaminomaleinsäurenitril der Formel (IV)

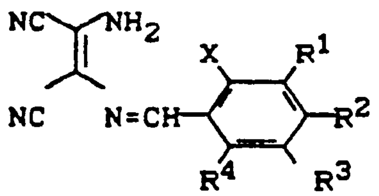

(IV)

in welcher

X, $R^1$, $R^2$, $R^3$ und $R^4$ die obengenannte Bedeutung haben, in einem geeigneten Lösungsmittel reduziert.

Schließlich wurde gefunden, daß die Benzylverbindungen der Formel ($I_C$) stark ausgeprägte akarizide Eigenschaften besitzen.

Überraschenderweise zeigen die Benzylverbindungen der Formel ($I_C$) eine akarizide Wirksamkeit. Eine derartige Wirkung ist von der Stoffklasse der Benzylverbindungen des Diaminomaleinsäurenitrils bisher noch nicht bekannt geworden. Die erfindungsgemaßen Verbindungen stellen somit eine Bereicherung des Standes der Technik dar.

Die Verbindungen der allgemeinen Formel ($I_C$) können in den stereoisomeren cis- und/oder trans-Formen vorliegen, z.B.

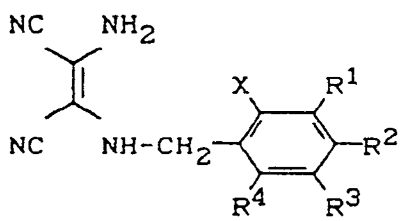

(cis)

bzw.

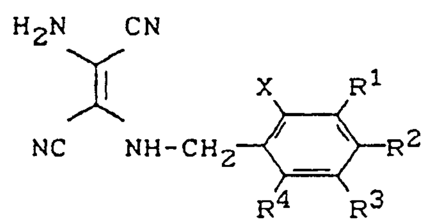

(trans)

Vorzugsweise liegen die Verbindungen jedoch in der cis-Form vor.

In der Formel ($I_C$) steht X vorzugsweise für Fluor, Chlor, Brom, Iod, $CF_3$ oder CN.

Bevorzugt sind Verbindungen der Formel ($I_C$)

in welcher

X und $R^4$ gleich oder verschieden sein können und für Fluor, Chlor, Brom, Iod, $CF_3$ oder CN stehen,

$R^1$, $R^2$ und $R^3$ gleich oder verschieden sein können und für Wasserstoff, Alkyl($C_1$-$C_4$), Halogenalkyl($C_1$-$C_4$), Alkoxy($C_1$-$C_4$), Halogenalkoxy($C_1$-$C_4$), Halogen, CN, $NO_2$, Dialkyl($C_1$-$C_4$)amino, Alkoxy($C_1$-$C_4$)carbonyl, Alkyl($C_1$-$C_4$)thio, Alkyl($C_1$-$C_4$)thionyl, Dihalogenalkyl($C_1$-$C_4$)amino, Alkyl($C_1$-$C_4$)sulfonyl, OH, SH, $NH_2$ stehen.

Besonders bevorzugt sind Verbindungen der allgemeinen Formel ($I_C'$)

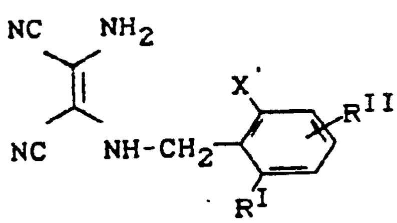

($I_C$),

in der

$X'$ und $R^I$ gleich oder verschieden sein können und für Fluor, Chlor, Brom, Iod oder $CF_3$ stehen

und

$R^{II}$ für Wasserstoff, Chlor, Brom, Iod, Cyan, Nitro, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Pentafluorethyl, Trifluorethyl, Trifluormethylsulfon, Trifluormethoxy, Trichlormethoxy, Pentafluorethoxy, Dimethylamino, Diethylamino, Di-β-chlorethylamino, Di-β-hydroxyethylamino steht.

Ganz besonders bevorzugt sind Verbindungen der Formel ($I_{C''}$)

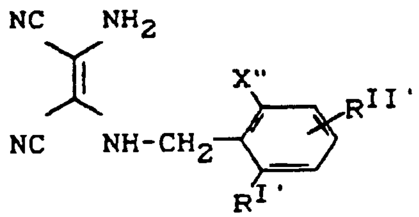

($I_{C''}$)

in der

$X''$ und $R^{I'}$ gleich oder verschieden sein können und für Chlor oder Brom stehen

und

$R^{II'}$ für Chlor, Brom, Iod, Wasserstoff, Cyan, Nitro, Methyl, Ethyl, n- und i-Propyl, Methoxy, Ethoxy, Methoxycarbonyl, Ethoxycarbonyl, Trifluormethyl, Trichlormethyl, Dimethylamino, Diethylamino, Trifluormethylsulfon steht.

Von ganz besonderem Interesse sind Verbindungen der Formel ($I_{C'''}$)

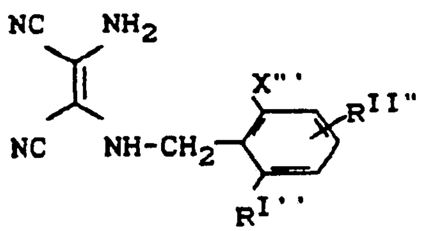

($I_{C'''}$)

in der

$X'''$ und $R^{I''}$ gleich oder verschieden sein können und für Chlor oder Brom stehen,

und

$R^{II''}$ für Wasserstoff, Chlor, Brom, Methyl, Cyano, Trichlormethyl, Trifluormethyl, Methoxy, Trifluormethoxy, Trichlormethoxy oder Trifluormethylsulfon steht.

Außerordentlich bevorzugt sind Verbindungen der allgemeinen Formel ($I_{C''''}$)

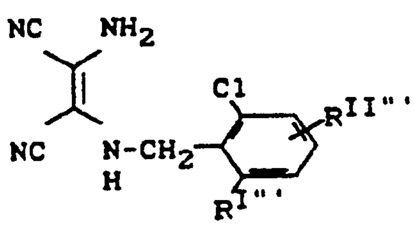

($I_{C''''}$)

in der

$R^{I'''}$ für Chlor oder Brom steht und

$R^{II'''}$ für Wasserstoff, Chlor, Methyl, Trichlormethyl, Methoxy, Trichlormethoxy, Trifluormethoxy steht.

Als außergewöhnlich bevorzugte erfindungsgemäß verwendete Verbindung der allgemeinen Formel ($I_C$) sei die Verbindung der Formel

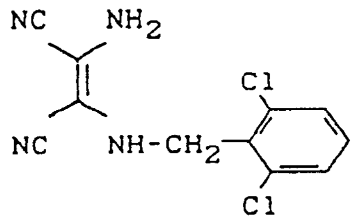

aufgeführt.

Die zur Durchführung des Herstellungsverfahrens als Ausgangsstoffe benötigten Azomethine der Formel (IV) können nach den in der Patentschrift JP 51 151 325 dargelegten Verfahren erhalten werden.

Als Verdünnungsmittel zur Durchführung des Herstellungsverfahrens kommen polare organische Lösungsmittel infrage, wie z.B. Alkohole (insbesondere Methanol, Ethanol, Propanol (n und i)), Dimethylformamid (DMF), Dimethylsulfoxid (DMSO), Hexamethylphosphorsäuretriamid (HMPT), als auch Acetonitril, Diethylether, Tetrahydrofuran.

Die Reaktionstemperatur liegt im allgemeinen im Bereich zwischen ca. 0°C bis maximal zum Siedepunkt des jeweiligen Lösungsmittels, insbesondere wird die Reaktion bei Temperaturen von ca. 10 bis ca. 50°C durchgeführt.

Die Umsetzung wird vorzugsweise bei Normaldruck durchgeführt.

Als Reduktionsmittel finden Metallhydride Verwendung z.B. des Aluminiums, Zinns und Bors.

Bevorzugt ist der Einsatz von Natriumborhydrid in protischen Lösungsmitteln wie z.B. Methanol, Ethanol oder in aprotischen Lösungsmitteln wie z.B. DMF oder Hexamethylphosphorsäuretriamid (HMPT).

Die Aufarbeitung erfolgt nach üblichen Methoden, nach dem Austragen auf Eis bzw. Wasser wird das Produkt abgesaugt bzw. extrahiert.

Das Herstellungsverfahren zum Erhalt der Verbindung der Formel ($I_C$) kann wie folgt an einem Beispiel dargestellt werden :

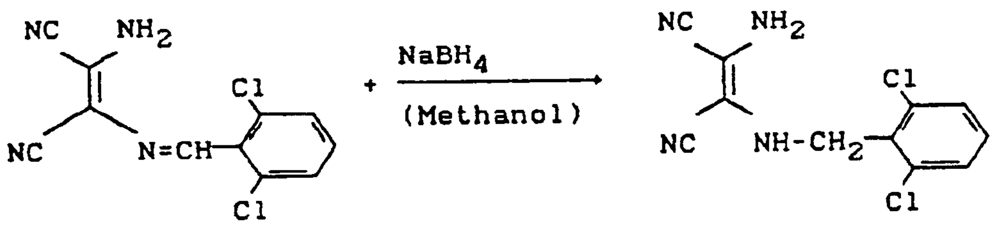


Im einzelnen seien außer den bei den Herstellungsbeispielen genannten Verbindungen die folgenden Benzyl-Derivate des 2,3-Diaminomaleinsäurenitrils der Formel ($I_C$) genannt :

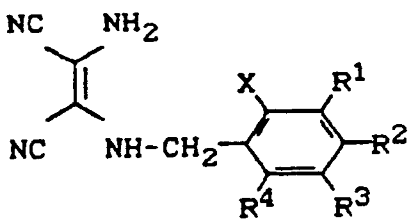

($I_C$)

| X | $R^1$ | $R^2$ | $R^3$ | $R^4$ |
|---|---|---|---|---|
| $CF_3$ | H | H | H | $CF_3$ |
| CN | H | H | H | H |
| Cl | H | H | H | H |
| H | Cl | H | H | H |
| H | H | Cl | H | H |
| Cl | H | F | H | Cl |
| Cl | H | Cl | H | Cl |
| Cl | H | Cl | Cl | H |
| Cl | H | H | H | $SCH_3$ |
| H | H | $N(CH_3)_2$ | H | H |
| H | Cl | H | Cl | OH |
| Cl | H | H | $NO_2$ | H |
| H | H | Br | H | H |
| F | F | F | F | F |
| H | H | F | Br | H |
| Cl | H | $CF_3$ | H | H |
| Cl | Cl | $CF_3$ | H | Cl |
| Cl | H | $COOCH_3$ | H | Cl |
| Cl | $CF_3$ | Cl | H | H |
| Br | H | Br | H | Br |
| Cl | H | Br | H | Cl |
| Cl | H | Cl | H | Br |
| Cl | H | $CF_3$ | H | Cl |
| Cl | H | $OCF_3$ | H | Cl |
| Cl | H | $OCCl_3$ | H | Cl |
| Cl | H | $OCF_3$ | H | H |
| Cl | H | $OCCl_3$ | H | H |
| Cl | H | $OCH_3$ | H | Cl |
| Cl | H | $OCH_3$ | H | H |

| X | $R^1$ | $R^2$ | $R^3$ | $R^4$ |
|---|---|---|---|---|
| Cl | H | $SCF_3$ | H | Cl |
| Cl | H | $SO_2CF_3$ | H | Cl |
| Cl | H | $SCF_3$ | H | H |
| Cl | H | $SO_2CF_3$ | H | H |
| Cl | H | $CH_3$ | H | H |
| Cl | H | H | H | $CH_3$ |
| Cl | H | $CF_3$ | H | $CH_3$ |
| Cl | H | $OCF_3$ | H | $CH_3$ |
| Cl | H | $SO_2CF_3$ | H | $CH_3$ |
| Cl | H | H | H | CN |
| Cl | H | CN | H | Cl |
| H | $OCH_3$ | OH | H | H |
| H | H | H | $OCH_3$ | OH |
| H | H | Cl | $NO_2$ | H |
| H | H | H | H | $NO_2$ |
| H | $NO_2$ | H | H | H |
| Br | H | H | H | Cl |
| H | H | $NO_2$ | H | H |
| H | Cl | $CF_3$ | H | H |
| H | Br | OH | $OCH_3$ | H |
| Br | H | H | H | Br |
| Br | H | $CH_3$ | H | H |
| $CF_3$ | H | F | H | $CF_3$ |
| Cl | H | H | Cl | H |
| Cl | H | Cl | OH | Cl |
| Cl | Cl | OH | Cl | H |
| F | F | OH | F | F |
| H | J | OH | J | H |

| X | $R^1$ | $R^2$ | $R^3$ | $R^4$ |
|---|---|---|---|---|
| Cl | H | H | H | $SO_2CH_3$ |
| Cl | H | H | H | $SO_2CF_3$ |
| H | H | CN | H | H |
| Br | H | CN | H | Br |
| Cl | H | CN | H | Cl |
| H | H | $N(CH_2CH_2CN)_2$ | H | H |
| H | H | $N(CH_2CH_2Cl)_2$ | H | H |

Man erhält die neuen Bisbenzyl-Derivate des 2,3-Diaminomaleinsäurenitrils der Formel ($I_D$)

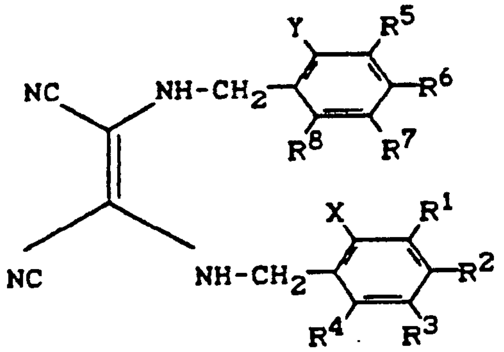

($I_D$)

in welcher X, Y, $R^1$, $R^2$, $R^3$, $R^4$, $R^5$, $R^6$, $R^7$ und $R^8$ die unter Formel ($I_A$) angegebene Bedeutung haben, wenn man die Bisanile der allgemeinen Formel ($I_A$)

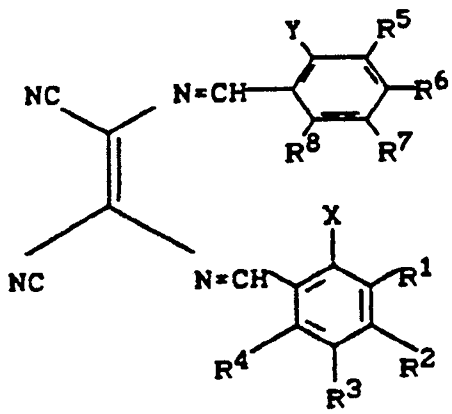

($I_A$)

oder die Benzyl-benzyliden-Derivate der allgemeinen Formel ($I_B$)

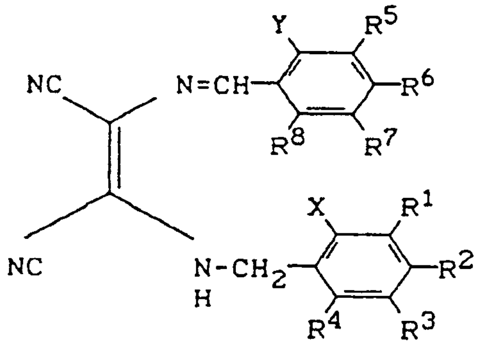

($I_B$)

in welcher X, Y, $R^1$, $R^2$, $R^3$, $R^4$, $R^5$, $R^6$, $R^7$ und $R^8$ unabhängig voneinander die obengenannte Bedeutung haben, in einem geeigneten Verdünnungsmittel reduziert.

Die Verbindungen der allgemeinen Formel ($I_D$) können in den stereoisomeren cis- und/oder trans-Formen vorliegen, Vorzugsweise liegen die Verbindungen jedoch in der cis-Form vor.

Die akarizid wirksamen Bisbenzyl-Verbindungen sind durch die Formel ($I_D$) allgemein definiert.

In der Formel ($I_D$) stehen X und Y vorzugsweise für Wasserstoff, Fluor, Chlor, Brom, Iod, $CF_3$ oder CN.

Bevorzugt sind Verbindungen der Formel ($I_D$)

in welcher

X und $R^4$ sowie Y und $R^8$ gleich oder verschieden sein können und für Fluor, Chlor, Brom, Iod, $CF_3$ oder CN stehen,

$R^1$, $R^2$ und $R^3$ sowie $R^5$, $R^6$, $R^7$ gleich oder verschieden sein können und für Wasserstoff, Alkyl($C_1$-$C_4$), Halogenalkyl($C_1$-$C_4$), Alkoxy($C_1$-$C_4$), Halogenalkoxy($C_1$-$C_4$), Halogen, CN, $NO_2$, Dialkyl($C_1$-$C_4$)amino, Alkoxy($C_1$-$C_4$)carbonyl, Alkyl($C_1$-$C_4$)thio, Alkyl($C_1$-$C_4$)thionyl, Dihalogenalkyl($C_1$-$C_4$)amino, Alkyl($C_1$-$C_4$)sulfonyl, OH, SH, $NH_2$ stehen.

Besonders bevorzugt sind Verbindungen der allgemeinen Formel ($I_{D'}$)

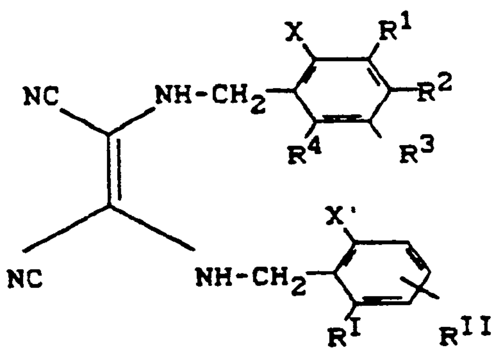

($I_{D'}$)

in der

X, $R^1$, $R^2$, $R^3$ und $R^4$ die oben angegebene Bedeutung haben und

X' und $R^I$ gleich oder verschieden sein können und für Fluor, Chlor, Brom, Iod oder $CF_3$ stehen und

$R^{II}$ für Wasserstoff, Chlor, Brom, Iod, Cyan, Nitro, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Pentafluorethyl, Trifluorethyl, Trifluormethylsulfon, Trifluormethoxy, Trichlormethoxy, Pentafluorethoxy, Dimethylamino, Diethylamino, Di-β-chlorethylamino, Di-β-hydroxyethylamino steht.

Ganz besonders bevorzugt sind verbindungen der Formel ($I_{D''}$)

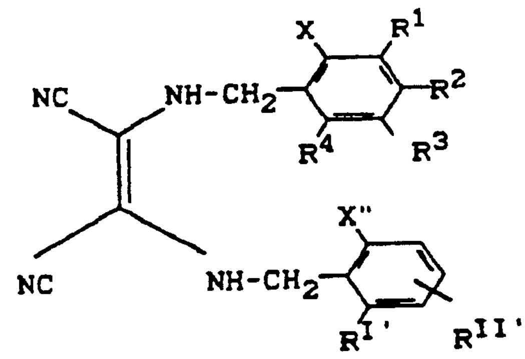

$(I_{D''})$

in der

$X''$ und $R^{I'}$ gleich oder verschieden sein können und für Chlor oder Brom stehen

und

$R^{II'}$ für Chlor, Brom, Iod, Wasserstoff, Cyan, Nitro, Methyl, Ethyl, n- und i-Propyl, Methoxy, Ethoxy, Methoxycarbonyl, Ethoxycarbonyl, Trifluormethyl, Trichlormethyl, Dimethylamino, Diethylamino, Trifluormethylsulfon steht.

Außerordentlich bevorzugt sind Verbindungen der Formel $(I_{D'''})$

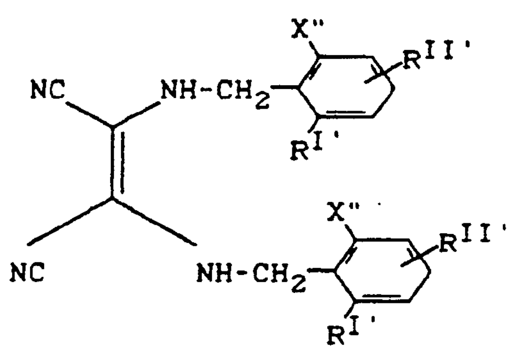

$(I_{D'''})$

in der

$X''$ und $R^{I'}$ gleich oder verschieden sein können und für Chlor oder Brom stehen

und

$R^{II'}$ für Chlor, Brom, Iod, Wasserstoff, Cyan, Nitro, Methyl, Ethyl, n- und i-Propyl, Methoxy, Ethoxy, Methoxycarbonyl, Ethoxycarbonyl, Trifluormethyl, Trichlormethyl, Dimethylamino, Diethylamino, Trifluormethylsulfon steht.

Von ganz besonderem Interesse weiterhin sind Verbindungen der Formel $(I_{D''''})$

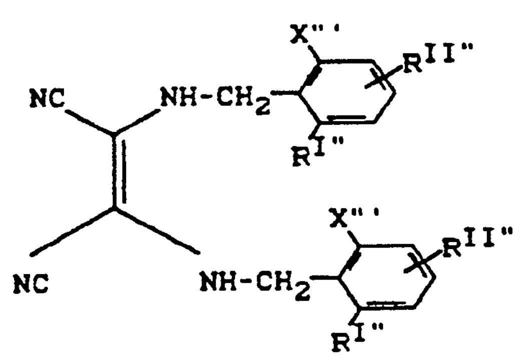

$(I_{D''''})$

in der

$X'''$ und $R^{I''}$ für Chlor oder Brom steht und

$R^{II''}$ für Wasserstoff, Chlor, Methyl, Trifluormethyl, Methoxy, Trichlormethyl, Ethoxy, Trichlormethoxy oder Trifluormethoxy steht.

Als Verdünnungsmittel zur Durchführung des erfindungsgemäßen Verfahrens kommen sowohl polare organische Lösungsmittel infrage, wie z.B. Alkohole (insbesondere Methanol, Ethanol, Propanol (n und i)), Dimethylformamid (DMF), Dimethylsulfoxid (DMSO), Hexamethylphosphorsäuretriamid (HMPT), als auch Acetonitril, Diethylether, Tetrahydrofuran.

Die Reaktionstemperatur liegt im allgemeinen im Bereich zwischen ca. 0°C bis maximal zum Siedepunkt des jeweiligen Lösungsmittels, insbesondere wird die Reaktion bei Temperaturen von ca. 10 bis ca. 50°C durchgeführt.

Die Umsetzung wird vorzugsweise bei Normaldruck durchgeführt.

Als Reduktionsmittel finden vorzugsweise Metallhydride Verwendung z.B. Hydride des Aluminiums, Zinns und Bors.

Bevorzugt wird Natriumborhydrid in protischen Lösungsmitteln wie z.B. Methanol, Ethanol oder in aprotischen Lösungsmitteln wie z.B. DMF, Tetrahydrofuran oder Hexamethylphosphorsäuretriamid (HMPT).

Die Aufarbeitung erfolgt nach üblichen Methoden, im allgemeinen wird nach dem Austragen auf Eis bzw. Wasser das Produkt abgesaugt bzw. extrahiert.

Das Herstellungsverfahren zum Erhalt der Verbindungen ($I_D$) kann wie folgt an einem Beispiel dargestellt werden :

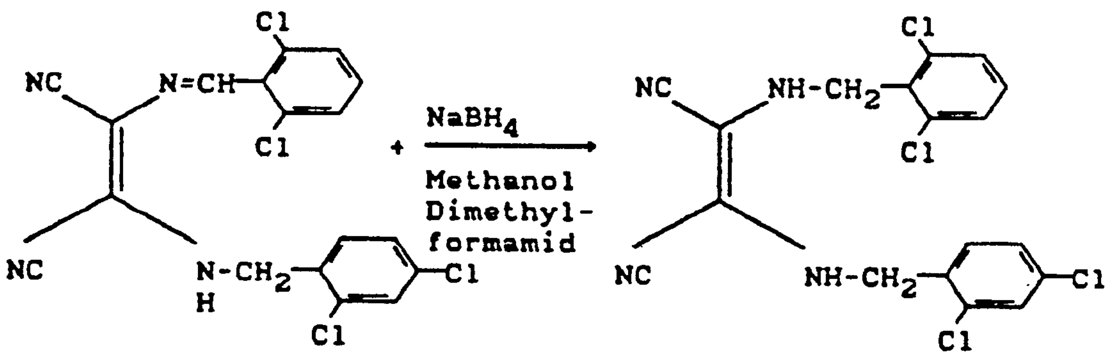


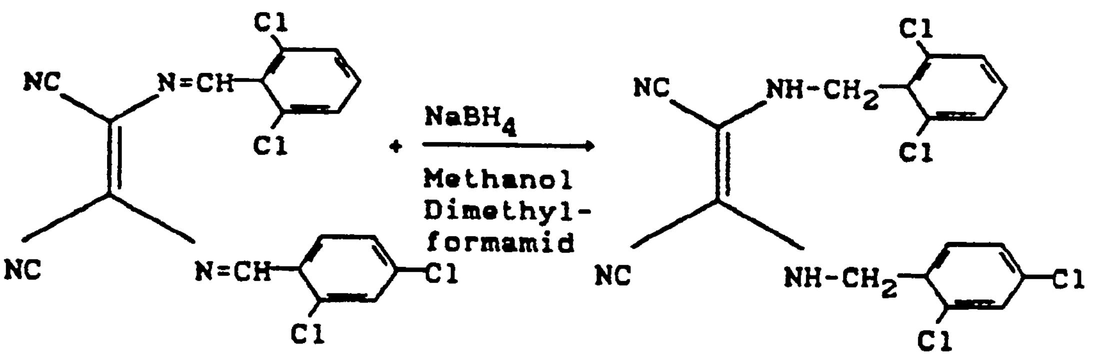


Im einzelnen seien außer den bei den Herstellungsbeispielen genannten Verbindungen die folgenden Bisbenzyl-Verbindungen des 2,3-Diaminomaleinsäurenitrils der Formel ($I_D$) genannt :

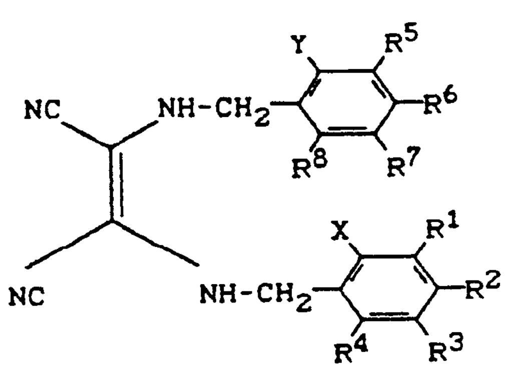

($I_D$)

| X | $R^1$ | $R^2$ | $R^3$ | $R^4$ | Y | $R^5$ | $R^6$ | $R^7$ | $R^8$ |
|---|---|---|---|---|---|---|---|---|---|
| H | H | H | H | H | Cl | H | H | H | H |
| H | H | H | H | H | Cl | H | Cl | H | H |
| H | H | H | H | H | Cl | H | Cl | H | Cl |
| H | H | H | H | H | Cl | H | H | H | F |
| H | H | H | H | H | H | $CF_3$ | H | H | H |
| H | H | H | H | H | $CF_3$ | H | H | H | $CF_3$ |
| H | H | H | H | H | Cl | $OCF_3$ | Cl | H | Cl |
| H | H | H | H | H | F | F | F | F | F |
| H | H | H | H | H | F | F | OH | F | F |
| H | H | H | H | H | $CF_3$ | H | F | H | $CF_3$ |
| H | H | H | H | H | Cl | H | F | H | Cl |
| H | H | H | H | H | Br | H | H | H | Cl |
| H | H | H | H | H | Cl | H | $CF_3$ | H | H |
| H | H | H | H | H | Cl | Cl | $CF_3$ | H | Cl |
| H | H | H | H | H | Cl | H | $COOCH_3$ | H | Cl |
| H | H | H | H | H | Cl | H | $OCH_3$ | H | Cl |
| H | H | H | H | H | Cl | H | $SCF_3$ | H | Cl |
| H | H | H | H | H | Cl | H | $SO_2CF_3$ | H | Cl |

| X | $R^1$ | $R^2$ | $R^3$ | $R^4$ | Y | $R^5$ | $R^6$ | $R^7$ | $R^8$ |
|---|---|---|---|---|---|---|---|---|---|
| H | H | H | H | H | Cl | H | $CH_3$ | H | H |
| H | H | H | H | H | Br | H | H | H | F |
| H | H | H | H | H | H | $OCH_3$ | OH | H | H |
| H | H | H | H | H | H | H | Cl | $NO_2$ | H |
| H | H | H | H | H | CN | H | H | H | Cl |
| H | H | H | H | H | H | H | CN | H | H |
| H | H | H | H | H | H | Br | OH | $OCH_3$ | H |
| Cl | H | H | H | H | Cl | H | H | H | Cl |
| Cl | H | H | H | H | Cl | H | Cl | H | H |
| Cl | H | H | H | H | Cl | H | $CH_3$ | H | H |
| Cl | H | H | H | H | CN | H | H | H | H |
| Cl | H | H | H | H | Br | H | Br | H | Br |
| Cl | H | H | H | H | Br | H | H | H | Cl |
| Cl | H | H | H | H | Cl | H | F | H | Cl |
| Cl | H | H | H | H | $CF_3$ | H | H | H | $CF_3$ |
| Cl | H | H | H | H | H | $CF_3$ | H | H | H |
| Cl | H | H | H | H | $CF_3$ | H | F | H | $CF_3$ |
| Cl | H | H | H | H | Cl | H | H | H | F |

| X | $R^1$ | $R^2$ | $R^3$ | $R^4$ | Y | $R^5$ | $R^6$ | $R^7$ | $R^8$ |
|---|---|---|---|---|---|---|---|---|---|
| Cl | H | H | H | H | Cl | $OCF_3$ | Cl | H | Cl |
| Cl | H | H | H | H | H | Cl | H | Cl | OH |
| H | Cl | H | H | H | Cl | H | H | H | Cl |
| H | Cl | H | H | H | Cl | H | Cl | H | H |
| H | Cl | H | H | H | Cl | H | $CH_3$ | H | H |
| H | Cl | H | H | H | CN | H | H | H | H |
| H | Cl | H | H | H | Br | H | H | H | Br |
| H | Cl | H | H | H | Br | H | Br | H | Br |
| H | Cl | H | H | H | Br | H | H | H | Cl |
| H | Cl | H | H | H | Cl | H | H | H | F |
| H | Cl | H | H | H | Cl | H | F | H | Cl |
| H | Cl | H | H | H | $CF_3$ | H | H | H | $CF_3$ |
| H | Cl | H | H | H | H | $CF_3$ | H | H | H |
| H | Cl | H | H | H | Cl | H | Cl | $OCF_3$ | Cl |
| H | Cl | H | H | H | H | Cl | H | Cl | OH |
| H | H | Cl | H | H | Cl | H | H | H | Cl |
| H | H | Cl | H | H | Cl | H | Cl | H | H |
| H | H | Cl | H | H | Cl | H | Cl | H | Cl |
| H | H | Cl | H | H | Cl | H | $CH_3$ | H | H |

| X | $R^1$ | $R^2$ | $R^3$ | $R^4$ | Y | $R^5$ | $R^6$ | $R^7$ | $R^8$ |
|---|---|---|---|---|---|---|---|---|---|
| H | H | Cl | H | H | CN | H | H | H | H |
| H | H | Cl | H | H | Br | H | H | H | Br |
| H | H | Cl | H | H | Br | H | Br | H | Br |
| H | H | Cl | H | H | Br | H | H | H | Cl |
| H | H | Cl | H | H | Cl | H | H | H | F |
| H | H | Cl | H | H | Cl | H | F | H | Cl |
| H | H | Cl | H | H | $CF_3$ | H | H | H | $CF_3$ |
| H | H | Cl | H | H | H | $CF_3$ | H | H | H |
| H | H | Cl | H | H | Cl | H | Cl | $OCF_3$ | Cl |
| H | H | Cl | H | H | H | Cl | H | Cl | OH |
| Cl | H | H | H | Cl | Cl | H | H | H | Cl |
| Cl | H | H | H | Cl | Cl | H | Cl | H | H |
| Cl | H | H | H | Cl | Cl | H | $CH_3$ | H | H |
| Cl | H | H | H | Cl | CN | H | H | H | H |
| Cl | H | H | H | Cl | Br | H | H | H | H |
| Cl | H | H | H | Cl | Br | H | Br | H | Br |
| Cl | H | H | H | Cl | Br | H | H | H | Cl |
| Cl | H | H | H | Cl | Cl | H | H | H | F |
| Cl | H | H | H | Cl | H | H | H | H | Cl |
| Cl | H | H | H | Cl | Br | H | H | H | Br |
| Cl | H | H | H | Cl | H | H | H | H | $CH_3$ |

| X | $R^1$ | $R^2$ | $R^3$ | $R^4$ | Y | $R^5$ | $R^6$ | $R^7$ | $R^8$ |
|---|---|---|---|---|---|---|---|---|---|
| Cl | H | H | H | Cl | Cl | H | F | H | Cl |
| Cl | H | H | H | Cl | $CF_3$ | H | H | H | $CF_3$ |
| Cl | H | H | H | Cl | H | $CF_3$ | H | H | H |
| Cl | H | H | H | Cl | Cl | H | Cl | $OCF_3$ | Cl |
| Cl | H | H | H | Cl | H | Cl | H | Cl | OH |
| Cl | H | Cl | H | H | Cl | H | H | H | Cl |
| Cl | H | Cl | H | H | Cl | H | Cl | H | H |
| Cl | H | Cl | H | H | Cl | H | $CH_3$ | H | H |
| Cl | H | Cl | H | H | CN | H | H | H | H |
| Cl | H | Cl | H | H | Br | H | H | H | Br |
| Cl | H | Cl | H | H | Br | H | Br | H | Br |
| Cl | H | Cl | H | H | Br | H | H | H | Cl |
| Cl | H | Cl | H | H | Cl | H | H | H | F |
| Cl | H | Cl | H | H | Cl | H | F | H | Cl |
| Cl | H | Cl | H | H | $CF_3$ | H | H | H | $CF_3$ |
| Cl | H | Cl | H | H | H | $CF_3$ | H | H | H |
| Cl | H | Cl | H | H | Cl | H | Cl | $OCF_3$ | Cl |
| Cl | H | Cl | H | H | H | Cl | H | Cl | OH |

| X | $R^1$ | $R^2$ | $R^3$ | $R^4$ | Y | $R^5$ | $R^6$ | $R^7$ | $R^8$ |
|---|---|---|---|---|---|---|---|---|---|
| H | $CF_3$ | H | H | H | Cl | H | H | H | Cl |
| H | $CF_3$ | H | H | H | Cl | H | Cl | H | H |
| H | $CF_3$ | H | H | H | Cl | H | $CH_3$ | H | H |
| H | $CF_3$ | H | H | H | CN | H | H | H | H |
| H | $CF_3$ | H | H | H | Br | H | H | H | Br |
| H | $CF_3$ | H | H | H | Br | H | Br | H | Br |
| H | $CF_3$ | H | H | H | Br | H | H | H | Cl |
| H | $CF_3$ | H | H | H | Cl | H | H | H | F |
| H | $CF_3$ | H | H | H | Cl | H | F | H | Cl |
| H | $CF_3$ | H | H | H | $CF_3$ | H | H | H | $CF_3$ |
| H | $CF_3$ | H | H | H | H | $CF_3$ | H | H | H |
| H | $CF_3$ | H | H | H | Cl | H | Cl | $OCF_3$ | Cl |
| H | $CF_3$ | H | H | H | H | Cl | H | Cl | OH |

| X | $R^1$ | $R^2$ | $R^3$ | $R^4$ | Y | $R^5$ | $R^6$ | $R^7$ | $R^8$ |
|---|---|---|---|---|---|---|---|---|---|
| Cl | H | H | H | F | Cl | H | H | H | Cl |
| Cl | H | H | H | F | Cl | H | Cl | H | H |
| Cl | H | H | H | F | Cl | H | $CH_3$ | H | H |
| Cl | H | H | H | F | CN | H | H | H | H |
| Cl | H | H | H | F | Br | H | H | H | Br |
| Cl | H | H | H | F | Br | H | Br | H | Br |
| Cl | H | H | H | F | Br | H | H | H | Cl |
| Cl | H | H | H | F | Cl | H | H | H | F |
| Cl | H | H | H | F | Cl | H | F | H | Cl |
| Cl | H | H | H | F | $CF_3$ | H | H | H | $CF_3$ |
| Cl | H | H | H | F | H | $CF_3$ | H | H | H |
| Cl | H | H | H | F | Cl | H | Cl | $OCF_3$ | Cl |
| Cl | H | H | H | F | H | Cl | H | Cl | OH |

| X | $R^1$ | $R^2$ | $R^3$ | $R^4$ | Y | $R^5$ | $R^6$ | $R^7$ | $R^8$ |
|---|---|---|---|---|---|---|---|---|---|
| $CF_3$ | H | H | H | $CF_3$ | Cl | H | H | H | H |
| $CF_3$ | H | H | H | $CF_3$ | H | Cl | Cl | H | H |
| $CF_3$ | H | H | H | $CF_3$ | H | $CF_3$ | H | H | H |
| $CF_3$ | H | H | H | $CF_3$ | H | Cl | H | Cl | OH |
| $CF_3$ | H | H | H | $CF_3$ | Cl | H | Cl | H | H |
| $CF_3$ | H | H | H | $CF_3$ | Cl | H | H | H | Cl |
| Cl | H | Cl | $OCF_3$ | Cl | Cl | H | H | H | H |
| Cl | H | Cl | $OCF_3$ | Cl | Cl | H | H | H | Cl |
| Cl | H | Cl | $OCF_3$ | Cl | Cl | H | Cl | H | H |
| Cl | H | Cl | $OCF_3$ | Cl | H | Cl | Cl | H | H |
| Cl | H | Cl | $OCF_3$ | Cl | H | $CF_3$ | H | H | H |
| Cl | H | Cl | $OCF_3$ | Cl | H | $OCH_3$ | OH | Br | H |
| H | Cl | Cl | H | H | Cl | H | H | H | Cl |

| X | $R^1$ | $R^2$ | $R^3$ | $R^4$ | Y | $R^5$ | $R^6$ | $R^7$ | $R^8$ |
|---|---|---|---|---|---|---|---|---|---|
| Cl | H | $CH_3$ | H | H | Cl | H | H | H | H |
| Cl | H | $CH_3$ | H | H | H | $CF_3$ | H | H | H |
| Cl | H | $CH_3$ | H | H | Cl | H | H | H | Cl |
| Cl | H | $CH_3$ | H | H | Cl | H | Cl | H | H |
| Cl | H | $CH_3$ | H | H | H | Cl | H | Cl | OH |
| Cl | H | $CF_3$ | H | Cl | Cl | H | Cl | H | H |
| Cl | H | $CF_3$ | H | Cl | H | $CF_3$ | H | H | H |
| Cl | H | $CF_3$ | H | Cl | Cl | H | H | H | Cl |
| Cl | H | $CF_3$ | H | Cl | H | Cl | H | Cl | OH |
| Cl | H | $CF_3$ | H | Cl | H | $NO_2$ | H | H | H |
| Cl | $CF_3$ | Cl | H | H | $CF_3$ | H | $CH_3$ | H | $CH_3$ |
| Cl | $CF_3$ | Cl | H | H | Cl | H | $CH_3$ | H | H |
| H | H | $N(CH_3)_2$ | H | H | Cl | H | H | H | Cl |

Die Wirkstoffe eignen sich bei guter Pflanzenverträglichkeit und günstiger Warmblütertoxizität zur Bekämpfung von Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören :

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäß verwendbaren Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben.

Sie sind gegen normalsensible und resistente Arten und Stämme, sowie gegen alle parasitierenden und nicht parasitierenden Entwicklungsstadien der Ektoparasiten wirksam.

Die erfindungsgemäß verwendbaren Wirkstoffe zeichnen sich durch eine hohe akarizide Wirksamkeit aus. Sie lassen sich mit besonders gutem Erfolg gegen pflanzenschädigende Milben, wie beispielsweise gegen die gemeine Spinnmilbe (Tetranychus urticae) einsetzen.

Darüber hinaus weisen die erfindungsgemäß verwendbaren Verbindungen noch eine fungizide Wirksamkeit auf.

Die Wirkstoffe konnen in die üblichen Formulierungen übergeführt werden, wie Losungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel konnen z.B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid ; als feste Trägerstoffe kommen in Frage : z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier und/oder schaumerzeugende Mittel kommen in Frage : z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate ; als Dispergiermittel kommen in Frage : z.B. Lignin-Sulfitablaugen und Methylcellulose.

Es können in den Formulierungen Haft mittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäß verwendbaren Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden oder Fungiziden vorliegen. Zu den Insektiziden zählen beispielsweise Phosphorsäureester, Carbamate, Carbonsäureester, chlorierte Kohlenwasserstoffe,

Phenylharnstoffe, durch Mikroorganismen hergestellte Stoffe u.a.

Die erfindungsgemäß verwendbaren Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Die erfindungsgemäß verwendbaren Wirkstoffe eignen sich auch zur Bekämpfung von Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäß verwendbaren Wirkstoffe geschieht auf diesem Gebiet in bekannter Weise wie durch orale Anwendung in Form von beispielsweise Tabletten, Kapseln, Tränken, Granulaten, durch dermale bzw. äußerliche Anwendung in Form beispielsweise der Tauchens (Dippen), Sprühens (Sprayen) ; Aufgießens (pour-on and spot-on) und des Einpuderns sowie durch parenterale Anwnedung in Form beispielsweise der Injektion sowie ferner durch das "feed-through"-Verfahren. Daneben ist auch eine Anwendung als Formkörper (Halsband, Ohrmarke) möglich.

Beispiel A

Tetranychus-Test (resistent)

| | |
|---|---|
| Lösungsmittel : | 7 Gewichtsteile Dimethylformamid |
| Emulgator : | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnen spinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100%, daß alle Spinnmilben abgetötet wurden ; 0% bedeutet, daß keine Spinnmilben abgetötet wurden.

Bei diesem Test zeigen z.B. die folgenden Verbindungen der Herstellungsbeispiele eine starke Wirksamkeit gegen Spinnmilben : (3), (9), (12), (14), (15), (26).

## Tabelle A

(pflanzenschädigende Milben)

Tetranychus Test

| Wirkstoffe | Wirkstoff-konz. in % | Abtötungsgrad in % nach 7 Tagen |
|---|---|---|
| 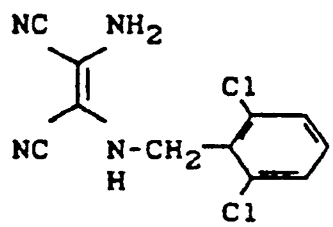 (3) | 0,1 | 98 |
| 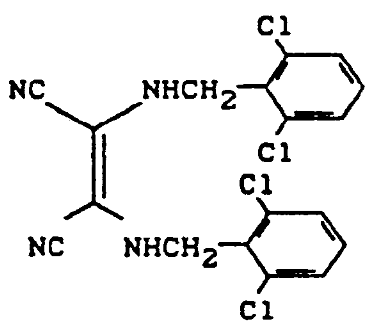 (26) | 0,1 | 98 |
| 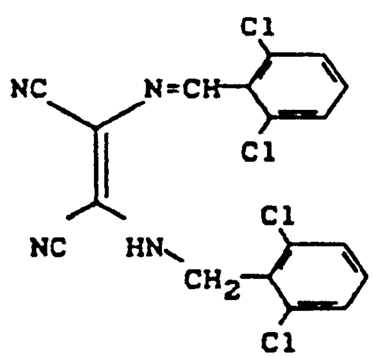 (9) | 0,1 | 98 |

Tabelle A
(pflanzenschädigende Milben)

Tetranychus Test

| Wirkstoffe | Wirkstoff-konz. in % | Abtötungsgrad in % nach 7 Tagen |
|---|---|---|
| 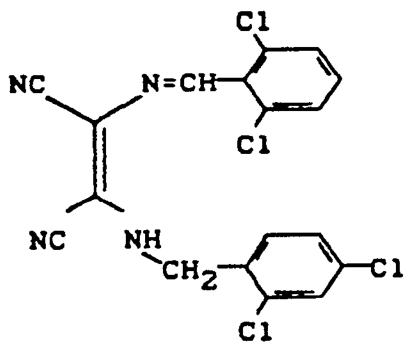 (14) | 0,1 | 98 |
| 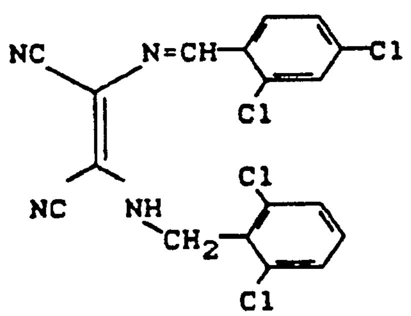 (15) | 0,1 | 98 |
| 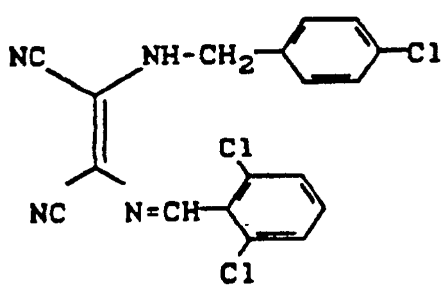 (12) | 0,1 | 98 |

Herstellungsbeispiele

Beispiel 1

13,25 g des N-2,6-Dichlorbenzyliden-amino-maleinsäurenitrils werden in 100 ml absolutem Ethanol mit 5,3 g Benzaldehyd und 100 mg p-Toluolsulfonsäure zum Rückfluß erhitzt. Man läßt noch 4 Stunden kochen und trägt dann auf 500 g Eis aus. Durch Absaugen isoliert man 13,0 g der folgenden Verbindung mit dem Schmelzpunkt > 250°C (Zers.) :

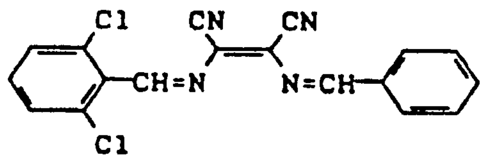

Beispiel 2

Verwendet man im obigen Beispiel 3-Trifluormethylbenzaldehyd anstelle des Benzaldehyds und wendet sonst die gleichen Reaktionsbedingungen an, so erhält man folgende Verbindung mit dem Schmelzpunkt 243°C.

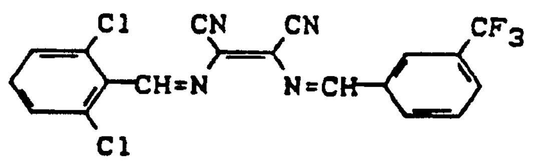

Beispiel 1A

Die in den Beispielen 1 und 2 eingesetzte Ausgangsverbindung N-2,6-Dichlorbenzyliden-amino-maleinsäurenitril der Formel

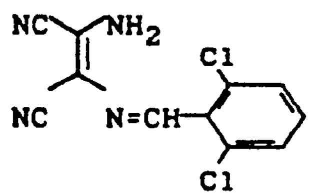

kann wie folgt hergestellt werden :

In 100 ml Acetonitril bringt man eine Mischung aus 10,8 g 2,3-Diaminomaleinsäurenitril und 17,5 g 2,6-Dichlorbenzaldehyd zum Sieden. Die Dünnschichtchromatographie zeigt nach 4 Stunden das Reaktionsende an. Nach dem Abkühlen saugt man 24,2 g (91% der Theorie) N-2,6-Dichlorbenzylidenaminomaleinsäurenitril ab. Die Substanz (gelbgrüne Nadeln) schmilzt bei 191°C.

Beispiel 3

In 200 ml Methanol bringt man eine Mischung aus 10,8 g 2,3-Diaminomaleinsäurenitril und 17,5 g 2,6-Dichlorbenzaldehyd zum Sieden. Die Dünnschichtchromatographie zeigt nach 4 Stunden die Bildung des Azomethins an. Anschließend kühlt man den Reaktionsansatz auf 0°C ab und gibt portionsweise 4,0 g Natriumborhydrid hinzu. Man läßt 30 Minuten nachrühren, bis eine klare braune Lösung erhalten wird. Nach dünsschichtchromatographischer Kontrolle trägt man den Ansatz auf 500 g Eis aus und saugt das ausgefallene Produkt ab. Man erhält 22,8 g der folgenden Verbindung

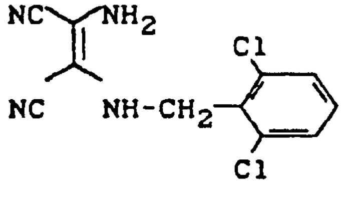

in Form eines gelbbraunen Pulvers vom Schmelzpunkt 159°C (aus Toluol).

Beispiel 4

In eine Lösung von 11,88 g 3-Trifluormethylbenzylidendiaminomaleinsäurenitril in 100 ml Methanol gibt man bei 0 bis 5°C 1,8 g Natriumborhydrid. Die Dünnschichtchromatographie zeigt das Reaktionsende an. Der Reaktionsansatz wird auf 500 g Eis ausgetragen und das Produkt abgesaugt. Man erhält 9,2 g eines Produkts der folgenden Formel

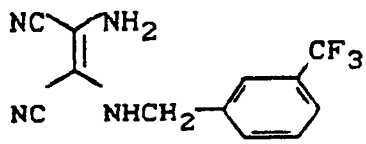

das nach Umkristallisieren aus wenig Chloroform bei 79°C schmilzt.

In Analogie zu den vorgenannten Beispielen werden die folgenden Verbindungen erhalten.

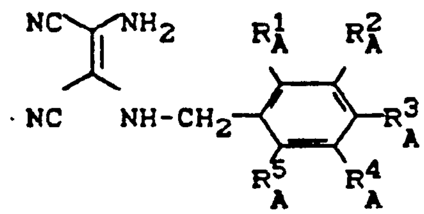

| Beispiel Nr. | $R_A^1$ | $R_A^2$ | $R_A^3$ | $R_A^4$ | $R_A^5$ | Schmelzpunkt |
|---|---|---|---|---|---|---|
| 5 | Cl | H | Cl | $OCF_3$ | Cl | 158° C |
| 6 | Cl | H | H | H | F | 141° C |
| 7 | H | H | H | H | H | 112° C |
| 8 | Cl | H | Cl | H | H | 122° C |

Beispiel 9

Zu 13,35 g des gemäß Beispiel 3 hergestellten N-2,6-Dichlorbenzyl-amino-maleinsäurenitrils in 100 ml Acetonitril gibt man 8,75 g 2,6-Dichlorbenzaldehyd und erhitzt zum Rückfluß. Das Azomethin kristallisiert schon in der Hitze aus. Man läßt abkühlen und saugt die gelben Kristalle ab. Man erhält 39,2 g der folgenden Verbindung mit dem Schmelzpunkt 132°C.

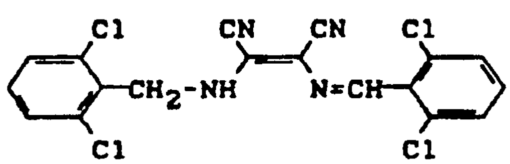

Beispiel 10

5,01 g des gemäß Beispiel 6 hergestellten N-2-Fluor-6-chlor-benzyl-amino-maleinsäurenitrils werden in 50 ml Acetonitril mit 3,5 g 2,6-Dichlorbenzaldehyd im Rückfluß erhitzt. Nach 4 Stunden kühlt man kräftig ab und saugt 6,7 g der folgenden Verbindung ab.

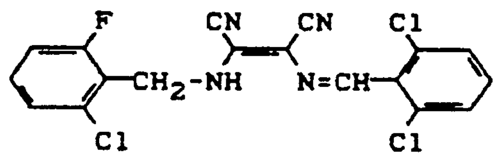

Sie schmilzt bei 159 bis 163°C.
Die folgenden Verbindungen werden in Analogie zu obigen Beispielen 9 und 10 erhalten.

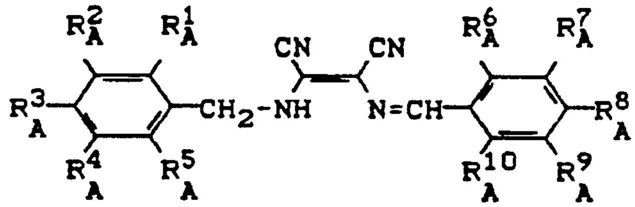

| Beispiel Nr. | $R^1_A$ | $R^2_A$ | $R^3_A$ | $R^4_A$ | $R^5_A$ | $R^6_A$ | $R^7_A$ | $R^8_A$ | $R^9_A$ | $R^{10}_A$ | Schmelzpunkt |
|---|---|---|---|---|---|---|---|---|---|---|---|
| 11 | Cl | H | H | H | Cl | H | $CF_3$ | H | H | H | 151° C |
| 12 | H | H | H | H | H | Cl | H | H | H | Cl | 126° C |
| 13 | H | H | H | H | H | H | H | $N(CH_3)_2$ | H | H | 195° C |
| 14 | Cl | H | Cl | H | H | Cl | H | H | H | Cl | 194° C |
| 15 | Cl | H | H | H | Cl | Cl | H | Cl | H | H | 152° C |
| 16 | H | Cl | H | H | H | OH | Cl | H | Cl | H | 217° C |
| 17 | Cl | H | H | H | H | Cl | H | Cl | $OCF_3$ | Cl | 128° C |
| 18 | H | Cl | H | H | H | H | Cl | Cl | H | H | 201° C |
| 19 | H | Cl | H | H | H | Cl | H | Cl | H | H | 193° C |
| 20 | H | Cl | H | H | H | Cl | H | H | H | Cl | 139° C |
| 21 | Cl | H | H | H | H | H | Cl | Cl | H | H | 174° C |
| 22 | Cl | H | H | H | H | Cl | H | Cl | H | H | 183° C |
| 23 | Cl | H | H | H | H | Cl | H | H | H | Cl | 164° C |
| 24 | Cl | H | H | H | Cl | H | Cl | Cl | H | H | 202° C |

Beispiel 25

21,2 g des gemäß Beispiel 14 hergestellten N-2,4-Dichlorbenzyl-N-2,6-Dichlorbenzyliden-maleinsäurenitrils werden in 100 ml Dimethylformamid und 300 ml Methanol gelöst und bei 0°C mit 2,0 g Natriumborhydrid versetzt. Man läßt anschließend noch 1 Stunde bei 25°C nachrühren. Der Reaktionsumsatz wird dünnschichtchromatographisch verfolgt. Die Aufarbeitung erfolgt durch Austragen auf 500 g Eis und Absaugen des Niederschlags. Man erhält 20,5 g der folgenden Verbindung vom Schmelzpunkt 101°C (aus Isopropanol).

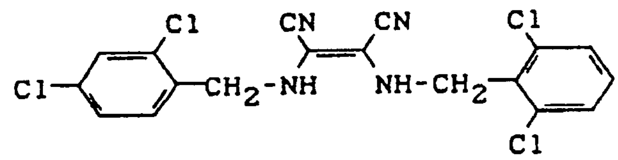

Beispiel 26

Nach dem oben beschriebenen Verfahren erhält man aus dem gemäß Beispiel 9 erhältlichen N-2,6-Dichlorbenzyl-N-2,6-dichlorbenzyliden-maleinsäurenitril durch Reduktion mit Natriumborhydrid die folgende Verbindung vom Schmelzpunkt 124°C (aus Isopropanol) :

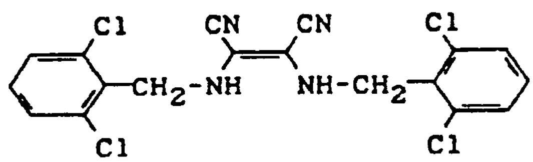

Formulierungsbeispiele

1. Stäubemittel

Zur Herstellung einer zweckmäßigen Wirkstoffzubereitung werden 5 Gew.-Teile Wirkstoff gemäß Beispiel 9 mit 95 Gew.-Teilen eines natürlichen Gesteinsmehl vermengt und staubfein vermahlen. Das so erhaltene Mittel wird in der jeweils gewünschten Menge durch verstäuben auf die Pflanzen oder ihren Lebensraum aufgebracht.

2. Spritzpulver (dispergierbares Pulver)

Zur Herstellung einer zweckmäßigen Wirkstoffzubereitung werden 50 Gew.-Teile Wirkstoff gemäß Beispiel 3 mit 1 Gew.-Teil Dibutylnaphthalinsulfonat, 4 Gew.-Teilen Ligninsulfonat, 8 Gew.-Teilen hochdisperser Kieselsäure sowie 37 Gew.-Teilen eines natürlichen Gesteinsmehles vermischt und zu einem Pulver vermahlen. Vor der Anwendung wird das benetzbare Pulver mit soviel Wasser verrührt, daß die dabei entstehende Mischung den Wirkstoff in der jeweils gewünschten Konzentration enthält.

3. Emulgierbares Konzentrat

Zur Herstellung einer zweckmäßigen Wirkstoffzubereitung werden 25 Gew.-Teile Wirkstoff gemäß Beispiel 14 in einer Mischung aus 55 Gew.-Teilen Xylol und 10 Gew.-Teilen Cyclohexanon gelöst. Als Emulgator setzt man anschließend 10 Gew.-Teile einer Mischung aus dodecylbenzolsulfonsaurem Calcium und Nonylphenolpolyglykolether hinzu.

Vor der Anwendung wird das Emulsionskonzentrat mit soviel Wasser verdünnt, daß die dabei entstehende Mischung den Wirkstoff in der jeweils gewünschten Konzentration enthält.

4. Granulat

Zur Herstellung einer zweckmäßigen Wirkstoffzubereitung gibt man zu 90 Gew.-Teilen Sand der Körnung 0,5 bis 1,0 mm 2 Gew.-Teile eines Spindelöls und anschließend 7 Gew.-Teile einer feingemahlenen Wirkstoff-

vermischung, die ihrerseits 75 Gew.-Teile Wirkstoff gemäß Beispiel 12 und 25 Gew.-Teile natürliches Gesteinsmehl enthält. Die Mischung wird so lange in einem geeigneten Mischer behandelt, bis ein gleichmäßiges, frei fließendes und nicht staubendes Granulat entstanden ist. Das Granulat wird in der jeweils gewünschten Menge auf die Pflanzen oder deren Lebensraum verstreut.

## Ansprüche

1. Verwendung von Derivaten des 2,3-Diaminomaleinsäurenitrils der allgemeinen Formel

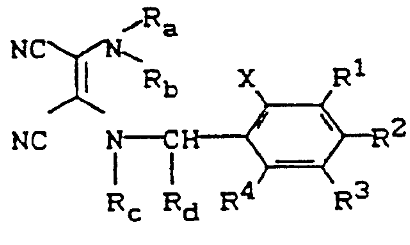

(I)

zur Bekämpfung von Spinnentieren (Pflanzen-, Hygieneund Vorratsschädlinge), wobei der Formel (I)

X für Wasserstoff, Halogen, Halogenalkyl oder CN steht,

$R^1$, $R^2$, $R^3$ und $R^4$ gleich oder verschieden sein können und für Wasserstoff, gegebenenfalls durch Halogen, OH, $NH_2$ substituiertes ($C_1$-$C_4$)Alkyl, gegebenenfalls durch Halogen, OH, $NH_2$ substituiertes ($C_1$-$C_4$)Alkoxy, Halogen, CN, $NO_2$, gegebenenfalls durch Halogen, OH, $NH_2$ substituiertes Dialkyl ($C_1$-$C_4$)amino, Alkoxy($C_1$-$C_4$)carbonyl, gegebenenfalls durch Halogen, OH, $NH_2$ substituiertes Alkyl($C_1$-$C_4$)thio, gegebenenfalls durch Halogen, OH, $NH_2$ substituiertes Alkyl($C_1$-$C_4$)sulfonyl, OH, SH, $NH_2$ stehen.

und die Substituenten $R_a$, $R_b$, $R_c$ und $R_d$ jeweils eine der folgenden Bedeutungskombinationen A, B, C oder D annehmen können :

A) $R_a$ und $R_b$ stehen zusammen für den Rest

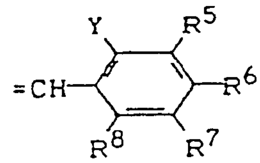

wobei

Y und $R^8$ gleich oder verschieden sein können und für Wasserstoff, Fluor, Chlor, Brom, Jod, $CF_3$ oder CN steht,

$R^5$, $R^6$ und $R^7$ gleich oder verschieden sein können und für Wasserstoff, gegebenenfalls durch Halogen, OH, $NH_2$ substituiertes ($C_1$-$C_4$)Alkyl, gegebenenfalls durch Halogen, OH, $NH_2$ substituiertes ($C_1$-$C_4$)Alkoxy, Halogen, CN, $NO_2$, gegebenenfalls durch Halogen, OH, $NH_2$ substituiertes Dialkyl($C_1$-$C_4$)amino, Alkoxy($C_1$-$C_4$)carbonyl, gegebenenfalls durch Halogen, OH, $NH_2$ substituiertes Alkyl($C_1$-$C_4$)thio, gegebenenfalls durch Halogen, OH, $NH_2$ substituiertes Alkyl($C_1$-$C_4$)thionyl, gegebenenfalls durch Halogen, OH, $NH_2$, substituiertes Alkyl($C_1$-$C_4$)sulfonyl, OH, SH, $NH_2$ stehen

und wobei $R_c$ und $R_d$ zusammen für eine chemische Bindung stehen,

B) $R_a$ und $R_b$ stehen zusammen für den Rest

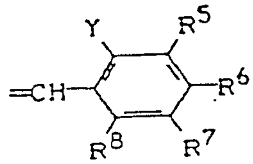

stehen,

wobei Y, $R^5$, $R^6$, $R^7$ und $R^8$ die unter A) angegebene Bedeutung besitzen und wobei $R_c$ und $R_d$ jeweils für

Wasserstoff stehen,

C) $R_a$, $R_b$, $R_c$ und $R_d$ stehen jeweils für Wasserstoff,

D) $R_a$ steht für Wasserstoff und $R_b$ steht für den Rest

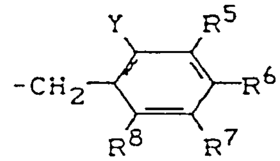

wobei Y, $R^5$, $R^6$, $R^7$ und $R^8$ die unter A) angegebene Bedeutung besitzen und $R_d$ and $R_d$ für Wasserstoff stehen.

2. Verfahren zur Bekämpfung von Spinnentieren (Pflanzen-, Hygiene- und Vorratsschädlinge) dadurch gekennzeichnet, daß man Derivate des 2,3-Diamino-maleinsäurenitrils der allgemeinen Formel (I) gemäß Anspruch 1 auf Spinnentiere und/oder ihren Lebensraum einwirken läßt.

## Claims

1. Use of derivatives of 2,3-diaminomaleonitrile of the general formula

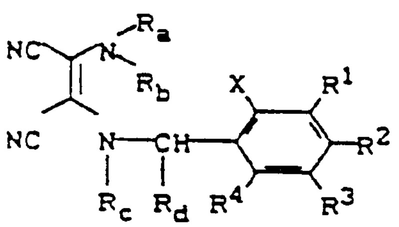

(I)

in which, in the formula I for combating arachnida (plant, hygiene, and stored product pests)

X represents hydrogen, halogen, halogenoalkyl or CN,

$R^1$, $R^2$, $R^3$ and $R^4$ can be identical or different and represent hydrogen, optionally halogen-, OH- or $NH_2$-substituted ($C_1$-$C_4$)alkyl, optionally halogen-, OH- or $NH_2$-substituted ($C_1$-$C_4$)alkoxy, halogen, CN, $NO_2$, optionally halogen-, OH- or $NH_2$-substituted dialkyl ($C_1$-$C_4$)amino, alkoy ($C_1$-$C_4$)carbonyl, optionally halogen-, OH- or $NH_2$-substituted alkyl($C_1$-$C_4$)thio, optionally halogen-, OH- or $NH_2$-substituted alkyl($C_1$-$C_4$)sulphonyl, OH, SH or $NH_2$

and the substituents $R_a$, $R_b$, $R_c$ and $R_d$ can in each case assume one of the following combinations of meaning A, B, C or D :

A) $R_a$ and $R_b$ together represent the radical

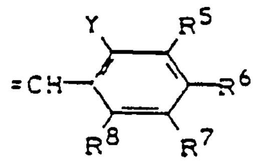

in which

Y and $R^8$ can be identical or different and represent hydrogen, fluorine, chlorine, bromine, iodine, $CF_3$, or CN,

$R^5$, $R^6$, and $R^7$ can be identical or different and represent hydrogen, optionally halogen-, OH- or $NH_2$-substituted($C_1$-$C_4$)alkyl, optionally halogen-, OH- or $NH_2$- substituted ($C_1$-$C_4$)alkoxy, halogen, CN, $NO_2$-, optionally halogen-, OH- or $NH_2$-substituted dialkyl($C_1$-$C_4$)amino, alkoxy($C_1$-$C_4$)carbonyl, optionally halogen-, OH- or $NH_2$-substituted alkyl($C_1$-$C_4$)thio, optionally halogen-, OH- or $NH_2$-substituted alkyl($C_1$-$C_4$)thionyl, optionally halogen-, OH- or $NH_2$-substituted alkyl($C_1$-$C_4$)sulphonyl, OH, SH or $NH_2$

and in which $R_c$ and $R_d$ together represent a chemical bond,

B) $R_a$ and $R_b$ together represent the radical

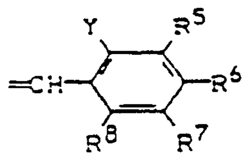

in which Y, $R^5$, $R^6$, $R^7$ and $R^8$ possess the meaning given under A) and in which $R_c$ and $R_d$ in each case represent hydrogen,

C) $R_a$, $R_b$, $R_c$ and $R_d$ in each case represent hydrogen,

D) $R_a$ represents hydrogen and $R_b$ represents the radical

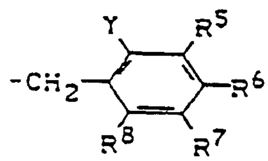

in which Y, $R^5$, $R^6$, $R^7$ and $R^8$ possess the meaning given under A) and $R_c$ and $R_d$ represent hydrogen.

2. Process for combating arachnida (plant, hygiene, and stored product pests), characterised in that derivatives of 2,3-diaminomaleonitrile of the general formula (I) according to Claim 1 are allowed to act on arachnida and/or their environment.

## Revendications

1. Utilisation de dérivés du nitrile d'acide 2,3-diaminomaléique de formule générale

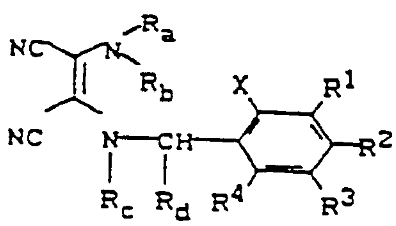

(I)

pour la lutte contre les arachnides (nuisibles aux plantes, à l'hygiène et aux réserves), les symboles de la formule ayant les significations suivantes :

X l'hydrogène, un halogène, halogénoalkyle ou CN,

$R^1$, $R^2$, $R^3$ et $R^4$ peuvent être identiques ou différents et représentent l'hydrogène, un groupe alkyle en $C_1$-$C_4$ éventuellement substitué par un halogène, un groupement OH, ou $NH_2$, un groupe alcoxy en $C_1$-$C_4$ éventuellement substitué par un halogène, un groupement OH ou $NH_2$, ou représentent un halogène, un groupe CN, $NO_2$, un groupe dialkyl ($C_1$-$C_4$)amino éventuellement substitué par un halogène, un groupement OH ou $NH_2$, un groupe alcoxy($C_1$-$C_4$)-carbonyle, un groupe alkyl($C_1$-$C_4$)thio éventuellement substitué par un halogène, un groupement OH ou $NH_2$, un groupe alkyl($C_1$-$C_4$)sulfonyle éventuellement substitué par un halogène, un groupement OH ou $NH_2$, ou représentent OH, SH, $NH_2$,

et les substituants $R_a$, $R_b$, $R_c$ et $R_d$ pouvant chacun avoir l'une des combinaisons de significations A, B, C ou D suivantes :

A) $R_a$ et $R_b$ représentent ensemble le reste

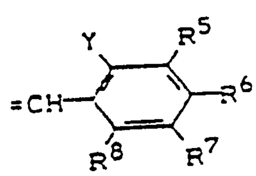

dans lequel

Y et $R^8$ peuvent être identiques ou différents et représentent l'hydrogène, le fluor, le chrome, le brome, l'iode, $CF_3$ ou CN,

$R^5$, $R^6$ et $R^7$ peuvent être identique ou différents et représentent l'hydrogène, un groupe alkyle en $C_1$-$C_4$ éventuellement substitué par un halogène, un groupement OH, $NH_2$, un groupe alcoxy en $C_1$-$C_4$ éventuellement substitué par un halogène, un groupement OH ou $NO_2$, un groupe dialkyl ($C_1$-$C_4$)amino éventuellement substitué par un halogène, un groupement OH ou $NH_2$, un groupe alcoxy($C_1$-$C_4$)-carbonyle, un groupe alkyl($C_1$-$C_4$)alkylthio éventuellement substitué par un halogène, un groupement OH ou $NH_2$, un groupe alkyl($C_1$-$C_4$)thionyle éventuellement substitué par un halogène, un groupement OH ou $NH_2$, un groupe alkyl($C_1$-$C_4$)sulfonyle éventuellement substitué par un halogène, un groupement OH ou $NH_2$, ou représentent OH, SH, $NH_2$,

et $R_c$ et $R_d$ représentent ensemble une liaison chimique,

B) $R_a$ et $R_b$ représentent ensemble le reste

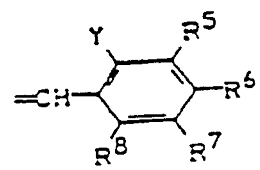

dans lequel Y, $R^5$, $R^6$, $R^7$ et $R^8$ possèdent les significations décrites en A) et $R_c$ et $R_d$ représentent chacun l'hydrogène,

C) $R_a$, $R_b$, $R_c$ et $R_d$ représentent chacun l'hydrogène,

D) $R_a$ représente l'hydrogène et $R_b$ représente le reste

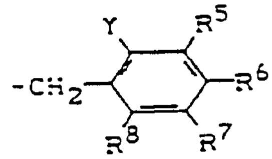

dans lequel Y, $R^5$, $R^6$, $R^7$ et $R^8$ possèdent les significations décrite en A) et $R_c$ et $R_d$ représentent l'hydrogène.

2. Procédé pour la lutte contre les arachnides (nuisibles aux plantes, à l'hygiène et aux réserves), caractérisé en ce que l'on fait agir des dérivés du nitrile d'acide 2,3-diamino-maléique de formule générale (I) selon la revendication 1 sur les arachnides et/ou sur leur habitat.